# EUROPEAN PATENT SPECIFICATION

(11) **EP 3 431 587 B1**
(45) Date of publication and mention of the grant of the patent: **26.10.2022**
(21) Application number: 17766465.3
(22) Date of filing: 07.03.2017
(51) Int. Cl.: C12N 5/077, C12N 15/09, C12N 5/0735, C12N 15/62, C12N 5/10

(54) **METHOD FOR PRODUCING CARDIAC PRECURSOR CELL AND MYOCARDIAL CELL FROM PLURIPOTENT STEM CELL**
VERFAHREN ZUR HERSTELLUNG EINER HERZVORLÄUFERZELLE UND MYOKARDIALEN ZELLE AUS PLURIPOTENTER STAMMZELLE
PROCÉDÉ DE PRODUCTION D'UNE CELLULE PRÉCURSEUR CARDIAQUE ET D'UNE CELLULE MYOCARDIQUE À PARTIR D'UNE CELLULE SOUCHE PLURIPOTENTE

(30) Priority: 15.03.2016 JP 2016051411
(43) Date of publication of application: 23.01.2019
(73) Proprietor: University of Tsukuba, Ibaraki 305-8577 (JP)
(72) Inventor: IEDA, Masaki, Tokyo 160-8582 (JP); SADAHIRO, Taketaro, Tokyo 160-8582 (JP); ISOMI, Mari, Tokyo 160-8582 (JP); GOSHIMA, Naoki, Tokyo 135-0064 (JP)
(74) Representative: Vossius & Partner Patentanwälte Rechtsanwälte mbB
(86) International application number: PCT/JP2017/009053
(87) International publication number: WO 2017/159464

(56) References cited:
- WO-A1-2010/008100
- JP-A- 2011 512 855
- JP-A- 2015 213 441
- JELLE VAN DEN AMEELE ET AL: "Eomesodermin induces Mesp1 expression and cardiac differentiation from embryonic stem cells in the absence of Activin", EMBO REPORTS, vol. 13, no. 4, 1 January 2012 (2012-01-01), pages 355-362, XP055031657, ISSN: 1469-221X, DOI: 10.1038/embor.2012.23
- LALIT PRATIK A ET AL: "Lineage Reprogramming of Fibroblasts into Proliferative Induced Cardiac Progenitor Cells by Defined Factors", CELL STEM CELL, vol. 18, no. 3, 3 March 2016 (2016-03-03), pages 354-367, XP029451693, ISSN: 1934-5909, DOI: 10.1016/J.STEM.2015.12.001
- TAKETARO SADAHIRO ET AL: "Tbx6 Induces Nascent Mesoderm from Pluripotent Stem Cells and Temporally Controls Cardiac versus Somite Lineage Diversification", CELL STEM CELL, vol. 23, no. 3, 1 September 2018 (2018-09-01), pages 382-395.e5, XP055620410, AMSTERDAM, NL ISSN: 1934-5909, DOI: 10.1016/j.stem.2018.07.001
- MARI ISOMI ET AL.: 'Shinki Shinzo Zenku Saibo Yudo Inshi ni yoru Hito ES /iPS karano Shinkin Yudoho no Kakuritsu' REGENERATIVE MEDICINE vol. 15, no. 2016, 01 February 2016,
- HIROYUKI YAMAKAWA ET AL.: 'FGF2/FGF10/VEGF ga, Shinkin Chokusetsu Full.Reprogramming o Koritsuteki ni Yudo suru' REGENERATIVE MEDICINE vol. 15, no. 2016, 01 February 2016, pages 213 , 0 - 11 -4
- GAVRILOV, S. ET AL.: 'Tbx6 is a determinant of cardiac and neural cell fate decisions in multipotent P19CL6 cells' DIFFERENTIATION vol. 84, no. 2, September 2012, pages 176 - 184, XP002722494

### SEQUENCE LISTING

<110> Keio University National Institute of Advanced Industrial Science and Technology
<120> Method of producing cardiac progenitor cells and cardiac myocytes from pluripotent stem cells
<130> G1526WO
<150> JP2016-051411
   <151> 2016-03-15
<160> 4
<170> PatentIn version 3.5
<210> 1
   <211> 1830
   <212> DNA
   <213> Homo sapiens
<400> 1
   agctgtcgga ctcaccgggg gccctaagca gcgagacctg aggccagacg gaactacaac        60
   atgtaccatc cacgagaatt gtacccgtcc ctgggggccg gctaccgcct ggggcccgcc       120
   caacctgggg ccgactccag cttcccaccc gccctagcgg agggctaccg ctaccccgaa       180
   ctggacaccc ctaaactgga ttgcttcctc tccgggatgg aggctgctcc ccgcaccctg       240
   gccgcgcacc cacctctgcc ccttctgccc cctgccatgg gcactgagcc ggccccatca       300
   gctccagagg ccctccattc cctcccgggg gtcagcctga gcctggagaa ccgggagcta       360
   tggaaggagt tcagctctgt gggaacagaa atgatcatca ccaaagctgg gaggcgcatg       420
   ttccctgcct gccgagtgtc agtcactggc ctggaccccg aggcccgcta cttgtttctt       480
   ctggatgtga ttccggtgga tggggctcgc taccgctggc agggccggcg ctgggagccc       540
   agcggcaagg cagagccccg cctgcctgac cgtgtctaca ttcaccccga ctctcctgcc       600
   actggtgcac attggatgcg gcagcctgtg tctttccatc gtgtcaagct caccaacagc       660
   acgctggacc cccacggcca cctgatcctg cactccatgc acaagtacca accccgcata       720
   cacctagttc gggcagccca gctctgcagc cagcactggg ggggcatggc ctccttccgc       780
   ttccccgaga ccacattcat ctccgtgaca gcctaccaga acccacagat cacacaactg       840
   aagattgcag ccaatccctt tgccaaaggc ttccgggaga acggcagaaa ctgtaagagg       900
   gagcgagacg cccgtgtgaa gaggaaactg cggggcccag agccagcagc cacagaggcc       960
   tatgggagcg gagacacacc aggtggtccc tgcgactcca ccctgggtgg agacattcgt      1020
   gaatcagatc cagaacaggc cccagccccc ggggaagcca ccgctgcccc ggcacctctg      1080
   tgtggtggcc ccagtgctga ggcctacctc ctgcaccctg cggctttcca tggggccccc      1140
   agtcaccttc ccaccaggag ccccagcttc ccggaggctc cagactccgg gcgctcagcc      1200
   ccctactcgg ctgcatttct ggagctgccg cacgggtcag gggctccggg gtacccagcg      1260
   gctccaccgg cggtaccctt tgccccgcac tttctccaag gggccccctt ccctctacca      1320
   tacaccgcgc ctgggggcta tctggatgtg ggctccaaac ccatgtactg aaccactgct      1380
   gggcccctct gcctccatca caaacctccc gctcccttcc cccagccctg gagccccctc      1440
   acctccaccg gccccatccc ccacaccaaa tggccgcctt gggcctgccc acccaccccc      1500
   tcaacttcac accttgattt cactcccacc cccctctggc ttcaaagcct aaccaaggct      1560
   gctgggagtc cagctggggc cggcttcccc ttcccggctc tcacctccgt gtgaatggaa      1620
   ggggctctgc ctggccaaat ggggccacag accagcatcc ccacttacca ggcctcccca      1680
   tgggctctgg aggggctcaa tccccacccc cccacacaca ctggtgcagg ccacaccagt      1740
   ctgttgttct gggaccagag tatttttgtt aataaaactc aaaagccatc catgctcagg      1800
   aaaaaaaaaa aaaaaaaaaa aaaaaaaaaa                                       1830
<210> 2
   <211> 436
   <212> **PRT**
   <213> Homo sapiens
<400> 2
<210> 3
   <211> 1731
   <212> DNA
   <213> Mus musculus
<400> 3
   cacaaggcca gaagaaacta caacatgtac catccacgag agttgtaccc ctccctgggg      60
   actggctacc gtctgggaca cccccagcct ggggcagact ccaccttccc acctgccctg     120
   acagagggtt accgctaccc tgatttggat acttctaaac tggattgctt cctctctggg     180
   atcgaggcag ctccccacac tctggctgca gccgctcctt tgccccttct cccatctgct     240
   ctgggccccg agacagcacc gccaccccca gaggcccttc actcgcttcc tggggtcagc     300
   ctgagcttgg agaaccagga actgtggaag gaattcagcg ctgtggggac agagatgatc     360
   atcaccaagg ctggcaggcg catgttccct gcttgccgag tatcagtcac tggcctggac     420
   ccagaggccc gctacttgtt tcttctggat gtggttccag tggatggggc ccgataccgc     480
   tggcagggcc agcactggga gccaagtggc aaggctgaac cccgcctacc cgaccgtgtc     540
   tacattcacc ctgactctcc tgccactggt gcccactgga tgcggcagcc cgtatccttc     600
   catcgtgtta agctcaccaa cagcacactg gacccccatg gccacctgat cttgcactcg     660
   atgcacaagt accagcctcg catccacctg gtgagagcca cccaactatg cagccaacac     720
   tgggggggtg tggcctcctt ccgatttcct gagaccacat tcatctctgt gacagcctac      780
   cagaacccta ggatcacaca gctgaagatc gcagccaatc cctttgccaa aggtttccga     840
   gaaaatggca gaaactgtaa gagggagcgg gatgcccgtg tgaagaggaa acttcggggc     900
   ccagagccag tggccacaga ggcctgtggg agtggggata caccaggggg tccctgtgac     960
   tccaccctgg gtggggacat tcgggactca gatccagagc aggccccaac cccccaggaa    1020
   gctgcttctg cctcagctcc tccatgtggg ggccccagtg ctgaggccta ccttctacac    1080
   cctgccgctt ttcatggcgc ccccagtcac ctaccagcca ggacccccag cttcgctgag    1140
   gctccagacc ctgggcgccc agccccctac tcagctgcat ttctggacct acagcctgga    1200
   ccagggggct ctgcctatca ggcagctcca tctgtaccat cctttgcccc acacttcatc    1260
   caagggggtc ccttccctct accgtaccca ggacctggag gttatctgga catgggatcc    1320
   aagccaatgt actgagcctc ggtgtagtaa ccctatgcca tcttcccttg atcctccagc    1380
   tcccttcccc cagcctggta gcatccgcat tgaagtggta tccccctccc ccccaccaaa    1440
   tggctggctt gggcctctct tccacccttt agttcacacc ttgatttcac tccaccccct    1500
   ctggcttcaa agctcaggca aggcagctct gagtccagct ggggcctgct tcccctcagc    1560
   tctcacctta gtgcgaatgg agggagcctc tgcctggcca aatggggctc ccagcccagt    1620
   acccccacct cctgggggcc ttgttactag tgcaagccat gccaatttgt tctcaggatc    1680
   agagtatttt tgttaataaa actccaaaga cattaaaaaa aaaaaaaaaa a             1731
<210> 4
   <211> 436
   <212> **PRT**
   <213> Mus musculus
<400> 4
   Met Tyr His Pro Arg Glu Leu Tyr Pro Ser Leu Gly Thr Gly Tyr Arg
   1               5                   10                  15
   Leu Gly His Pro Gln Pro Gly Ala Asp Ser Thr Phe Pro Pro Ala Leu
               20                  25                  30
   Thr Glu Gly Tyr Arg Tyr Pro Asp Leu Asp Thr Ser Lys Leu Asp Cys
           35                  40                  45
   Phe Leu Ser Gly Ile Glu Ala Ala Pro His Thr Leu Ala Ala Ala Ala
       50                  55                  60
   Pro Leu Pro Leu Leu Pro Ser Ala Leu Gly Pro Glu Thr Ala Pro Pro
   65                  70                  75                  80
   Pro Pro Glu Ala Leu His Ser Leu Pro Gly Val Ser Leu Ser Leu Glu
                   85                  90                  95
   Asn Gln Glu Leu Trp Lys Glu Phe Ser Ala Val Gly Thr Glu Met Ile
               100                 105                 110
   Ile Thr Lys Ala Gly Arg Arg Met Phe Pro Ala Cys Arg Val Ser Val
           115                 120                 125
   Thr Gly Leu Asp Pro Glu Ala Arg Tyr Leu Phe Leu Leu Asp Val Val
       130                 135                 140
   Pro Val Asp Gly Ala Arg Tyr Arg Trp Gln Gly Gln His Trp Glu Pro
   145                 150                 155                 160
   Ser Gly Lys Ala Glu Pro Arg Leu Pro Asp Arg Val Tyr Ile His Pro
                   165                 170                 175
   Asp Ser Pro Ala Thr Gly Ala His Trp Met Arg Gln Pro Val Ser Phe
               180                 185                 190
   His Arg Val Lys Leu Thr Asn Ser Thr Leu Asp Pro His Gly His Leu
           195                 200                 205
   Ile Leu His Ser Met His Lys Tyr Gln Pro Arg Ile His Leu Val Arg
       210                 215                 220
   Ala Thr Gln Leu Cys Ser Gln His Trp Gly Gly Val Ala Ser Phe Arg
   225                 230                 235                 240
   Phe Pro Glu Thr Thr Phe Ile Ser Val Thr Ala Tyr Gln Asn Pro Arg
                   245                 250                 255
   Ile Thr Gln Leu Lys Ile Ala Ala Asn Pro Phe Ala Lys Gly Phe Arg
               260                 265                 270
   Glu Asn Gly Arg Asn Cys Lys Arg Glu Arg Asp Ala Arg Val Lys Arg
           275                 280                 285
   Lys Leu Arg Gly Pro Glu Pro Val Ala Thr Glu Ala Cys Gly Ser Gly
       290                 295                 300
   Asp Thr Pro Gly Gly Pro Cys Asp Ser Thr Leu Gly Gly Asp Ile Arg
   305                 310                 315                 320
   Asp Ser Asp Pro Glu Gln Ala Pro Thr Pro Gln Glu Ala Ala Ser Ala
                   325                 330                 335
   Ser Ala Pro Pro Cys Gly Gly Pro Ser Ala Glu Ala Tyr Leu Leu His
               340                 345                 350
   Pro Ala Ala Phe His Gly Ala Pro Ser His Leu Pro Ala Arg Thr Pro
           355                 360                 365
   Ser Phe Ala Glu Ala Pro Asp Pro Gly Arg Pro Ala Pro Tyr Ser Ala
       370                 375                 380
   Ala Phe Leu Asp Leu Gln Pro Gly Pro Gly Gly Ser Ala Tyr Gln Ala
   385                 390                 395                 400
   Ala Pro Ser Val Pro Ser Phe Ala Pro His Phe Ile Gln Gly Gly Pro
                   405                 410                 415
   Phe Pro Leu Pro Tyr Pro Gly Pro Gly Gly Tyr Leu Asp Met Gly Ser
               420                 425                 430
   Lys Pro Met Tyr
           435

## Description

### Technical Field

The present invention relates to a method for producing cardiac progenitor cells and cardiomyocytes from ES cells or iPS cells. Moreover, there are disclosed cardiac progenitor cells and cardiomyocytes, which are produced by the aforementioned method.

### Background Art

Heart disease has steadily increased with aging, and the incidence of heart failure in men aged 80 or over is high (14.7%). The heart is composed of cells such as cardiomyocytes and fibroblasts. Since cardiomyocytes having a beating function have almost no or completely no regeneration ability, the method for treating heart disease has been restricted so far.

To date, a method for directly producing cardiomyocyte-like cells from fibroblasts, without going through iPS cells, wherein the method comprises introduction of three cardiac reprogramming factors (Gata4, Mef2c and Tbx5; hereinafter referred to as "GMT"), has been found (Non Patent Literature 1). It was elucidated that, according to this method, the cardiac muscle can be directly produced from fibroblasts by the three factors GMT even in cultured cells and in the living body of a mouse (Patent Literature 1). Moreover, it has been reported that functionally immature cardiomyocyte-like cells can be produced from fibroblasts through cardiac progenitor cells, by using transcriptional factors (Mesp1 and Ets2) and a plurality of humoral factors (Non Patent Literature 2). Furthermore, a method of inducing cardiac progenitor cells or cardiomyocytes from pluripotent stem cells such as ES cells or iPS cells, using a humoral factor (Non Patent Literature 3), and a method of inducing cardiac progenitor cells from mouse ES cells, using a transcriptional factor, under the use of serum or under special culture conditions, have been reported (Non Patent Literature 4).

On the other hand, a method of inducing cardiac progenitor cells and cardiomyocytes from pluripotent stem cells, using a humoral factor, has been problematic in terms of reproducibility and costs. Accordingly, if a stable pluripotent stem cell line that is induced to differentiate into the cardiac muscle could be produced by introduction of a transcriptional factor, it would be clinically useful.

Moreover, in conventional methods of inducing cardiac progenitor cells and cardiomyocytes from pluripotent stem cells, using a transcriptional factor, serum or special culture conditions have been used. There have been no reports regarding the use of a serum-free medium. Furthermore, conventional reports have related to only methods of inducing cardiac progenitor cells and cardiomyocytes from mouse cells, and there have been no reports regarding a method of inducing cardiac progenitor cells and cardiomyocytes from human cells.

### Citation List

### Patent Literature

Patent Literature 1: WO2011/139688

### Non Patent Literature

Non Patent Literature 1: Ieda, M., Fu, J.D., Delgado-Olguin, P., Vedantham, V., Hayashi, Y., Bruneau, B. G., and Srivastava, D. Direct Reprogramming of Fibroblasts into Functional Cardiomyocytes by Defined Factors. Cell 142: 375-386.2010.
Non Patent Literature 2: Islas JF, Liu Y, Weng KC, et al. Transcription factors ETS2 and MESP1 transdifferentiate human dermal fibroblasts into cardiac progenitors. Proceedings of the National Academy of Sciences of the United States of America 2012; 109(32): 13016-21.
Non Patent Literature 3: Kattman SJ, Witty AD, Gagliardi M, et al. Stagespecific optimization of activin/nodal and BMP signaling promotes cardiac differentiation of mouse and human pluripotent stem cell lines. Cell stem cell 2011; 8(2): 228-40.
Non Patent Literature 4: van den Ameele J, Tiberi L, Bondue A, et al. Eomesodermin induces Mesp1 expression and cardiac differentiation from embryonic stem cells in the absence of Activin. EMBO reports 2012; 13(4): 355-62.

### Summary of Invention

### Technical Problem

Under the aforementioned circumstances, it has been desired to develop a method of inducing cardiac progenitor cells from pluripotent stem cells under serum-free conditions by introduction of a transcriptional factor.

### Solution to Problem

As a result of intensive studies directed towards achieving the aforementioned object, the present inventors have introduced a Tbx6-expressing lentiviral vector capable of regulating the expression of Tbx6 by doxycycline into mouse ES cells and human iPS cells, so that the inventors have obtained stable pluripotent stem cells that are induced to differentiate into the cardiac muscle. Thereafter, the present inventors have found that the obtained pluripotent stem cells induced to differentiate into the cardiac muscle can be induced to differentiate into cardiac progenitor cells by turning the expression of Tbx6 on, under serum-free conditions and without using humoral factors and the like used in the conventional methods, and further that large quantities of cardiomyocytes are induced from the obtained cardiac progenitor cells by turning the expression of Tbx6 off.

The present invention has been completed based on these findings.

Specifically, the present invention is as follows.
[1] A method for producing cardiac progenitor cells from pluripotent stem cells, comprising expressing a Tbx6 gene in the pluripotent stem cells.
[2] A method for producing cardiomyocytes from pluripotent stem cells, comprising:
   (i) a step of inducing cardiac progenitor cells from pluripotent stem cells, comprising expressing a Tbx6 gene in the pluripotent stem cells, and
   (ii) a step of inducing cardiomyocytes from the cardiac progenitor cells induced in the step (i), comprising suppressing the expression of the Tbx6 gene.
[3] The method according to the above [1] or [2], wherein the expression of the Tbx6 gene and the suppression of the expression are carried out using an expression cassette that regulates the expression in response to external stimulation.
[4] The method according to any one of the above [1] to [3], wherein the step of expressing the Tbx6 gene in the pluripotent stem cells comprises:
   a step of introducing an expression cassette capable of inducing the expression of the Tbx6 gene in response to external stimulation, into the pluripotent stem cells; and
   a step of culturing the pluripotent stem cells, into which the expression cassette has been introduced, in the presence of external stimulation.
[5] The method according to any one of the above [1] to [4], wherein the step of expressing the Tbx6 gene in the pluripotent stem cells comprises:
   a step of introducing an expression cassette capable of inducing the expression of the Tbx6 gene in response to external stimulation, into the pluripotent stem cells; and
   a step of culturing the pluripotent stem cells, into which the expression cassette has been introduced, in the presence of external stimulation and in the absence of serum.
[6] The method according to any one of the above [2] to [5], wherein the step of suppressing the expression of the Tbx6 gene comprises:
   a step of culturing the pluripotent stem cells, into which the expression cassette capable of inducing the expression of the Tbx6 gene in response to external stimulation has been introduced, in the absence of external stimulation.
[7] The method according to any one of the above [1] to [6], wherein the pluripotent stem cells are ES cells or iPS cells.
[8] The method according to any one of the above [1] to [7], wherein the external factor is tetracycline or doxycycline.
   Moreover, in the context of the present invention, there is disclosed the following [9] and [10].
[9] A pluripotent stem cell, into which an expression cassette capable of inducing the expression of Tbx6 gene in response to external stimulation has been introduced, and which is induced to differentiate into a cardiac progenitor cell by being cultured in the presence of the external stimulation.
[10] A pluripotent stem cell, into which an expression cassette capable of inducing the expression of a Tbx6 gene in response to external stimulation has been introduced, and which is induced to differentiate into a cardiomyocyte by being cultured in the absence of the external stimulation after being cultured in the presence of the external stimulation.

Into the pluripotent stem cells according to the above [9] and [10], an expression cassette capable of inducing the expression of a Tbx6 gene in response to external stimulation has been introduced. Accordingly, the present pluripotent stem cells are cells comprising the aforementioned expression cassette.

### Advantageous Effects of Invention

According to the present invention, a method of inducing cardiac progenitor cells and cardiomyocytes from pluripotent stem cells is provided. In addition, pluripotent stem cells, which are induced to differentiate into cardiac progenitor cells and/or cardiomyocytes, are disclosed. The pluripotent stem cells of the present invention, which are induced to differentiate into the cardiac muscle, are able to regulate the induction of differentiation into cardiac progenitor cells and the induction of differentiation into cardiomyocytes by turning the expression of Tbx6 on and off, respectively.

More specifically, according to the present invention, when Tbx6 is expressed in the presence of external stimulation such as administration of doxycycline, by using a system capable of regulating the expression of a Tbx6 gene by the external stimulation, cardiac progenitor cells are induced from mouse ES cells or human iPS cells, and further, when the expression of Tbx6 is suppressed by removing the external stimulation, functionally mature cardiomyocytes are induced.

The method for producing cardiac progenitor cells and cardiomyocytes of the present invention has the efficiency of inducing cardiac progenitor cells and cardiomyocytes that is 2 to 3 times higher than that of Eomes, which has been previously reported regarding mouse cells. Accordingly, cardiac progenitor cells and cardiomyocytes can be produced with high efficiency according to the method for producing cardiac progenitor cells and cardiomyocytes of the present invention.

### Brief Description of Drawings

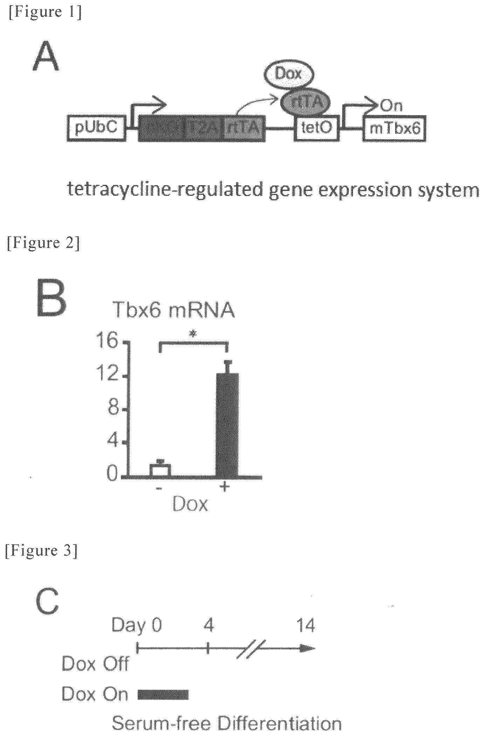

[Figure 1] Figure 1 is a schematic view showing a tetracycline-regulated gene expression system.
[Figure 2] Figure 2 is a view showing the expression level of Tbx6 in Tbx6 gene-introduced cells in the presence of doxycycline.
[Figure 3] Figure 3 is a schematic view showing the timing of adding doxycycline in Example 4.

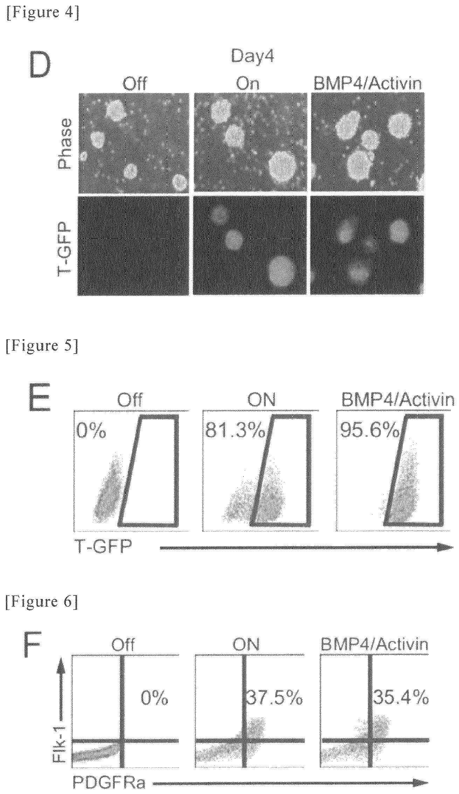

[Figure 4] Figure 4 is a view showing the GFP images in the cell line of Example 4 without addition of doxycycline ("Off"), the cell line of Example 4 in which Tbx6 has been induced by addition of doxycycline ("On"), and cardiac progenitor cells differentiated from ES cells with the use of BMP4 and Activin ("BMP4/Activin"), on Day 4 (Figure 3).
[Figure 5] Figure 5 is a view showing the expression of GFP in the cell line of Example 4 without addition of doxycycline ("Off'), the cell line of Example 4 in which Tbx6 has been induced by addition of doxycycline ("On"), and cardiac progenitor cells differentiated from ES cells with the use of BMP4 and Activin ("BMP4/Activin"), on Day 4 (Figure 3).
[Figure 6] Figure 6 is a view showing the expression of Flk-1 and PDGFRα in the cell line of Example 4 without addition of doxycycline ("Off"), the cell line of Example 4 in which Tbx6 has been induced by addition of doxycycline ("On"), and cardiac progenitor cells differentiated from ES cells with the use of BMP4 and Activin ("BMP4/Activin"), on Day 4 (Figure 3).

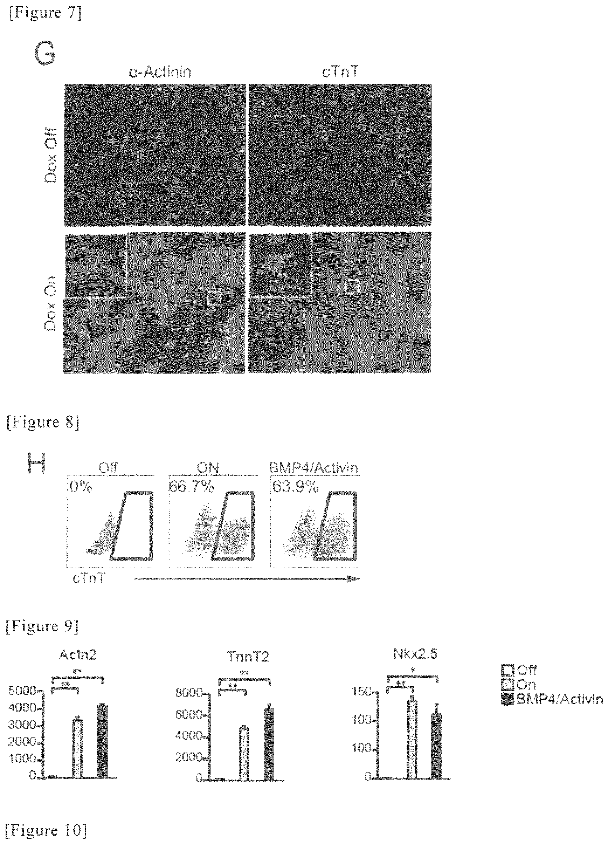

[Figure 7] Figure 7 is a view showing the images of α-actinin and cardiac troponin in the cell line of Example 4 without addition of doxycycline ("Dox Off") and the cell line of Example 4 in which Tbx6 has been induced by addition of doxycycline ("Dox On"), on Day 14 (Figure 3).
[Figure 8] Figure 8 is a view showing the expression of cardiac troponin in the cell line of Example 4 without addition of doxycycline ("Off'), the cell line of Example 4 in which Tbx6 has been induced by addition of doxycycline ("On"), and cardiac progenitor cells differentiated from ES cells with the use of BMP4 and Activin ("BMP4/Activin"), on Day 14 (Figure 3).
[Figure 9] Figure 9 is a view showing the expression levels of actinin (Actn2), cardiac troponin (TnnT2), and Nkx2.5 in the cell line of Example 4 without addition of doxycycline ("Off'), the cell line of Example 4 in which Tbx6 has been induced by addition of doxycycline ("On"), and cardiac progenitor cells differentiated from ES cells with the use of BMP4 and Activin ("BMP4/Activin"), on Day 14 (Figure 3).

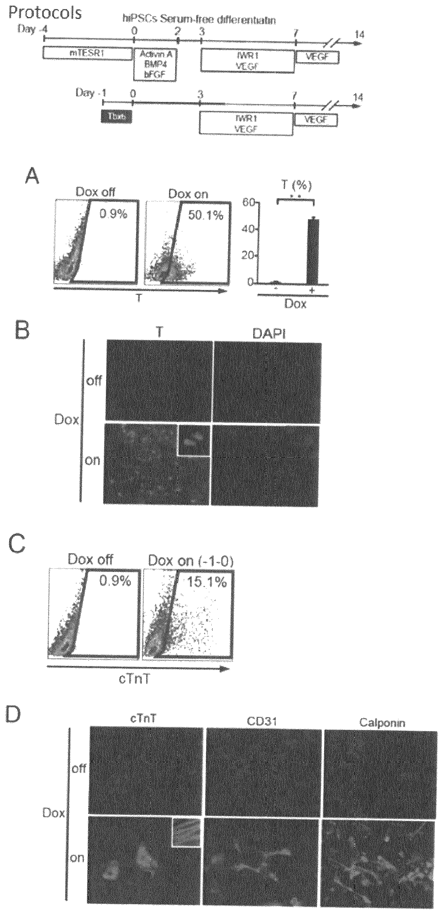

[Figure 10] Figure 10 is a view showing induction of human cardiac progenitor cells from human iPS cells and differentiation of the human progenitor cells into cardiomyocytes, according to the expression of Tbx6.

### Description of Embodiments

The present invention relates to pluripotent stem cells, which are induced to differentiate into cardiac progenitor cells and cardiomyocytes (pluripotent stem cells induced to differentiate into the cardiac muscle). The pluripotent stem cells induced to differentiate into the cardiac muscle of the present invention can control induction of differentiation into cardiac progenitor cells and/or induction of differentiation into cardiomyocytes, by turning the expression of Tbx6 on or off, in response to external stimulation.

The present invention relates to a method for producing cardiac progenitor cells from pluripotent stem cells, comprising expressing a Tbx6 gene in the pluripotent stem cells. In the present invention, by introducing an expression cassette that regulates the expression of a Tbx6 gene by external stimulation such as administration of doxycycline into pluripotent stem cells, the expression of a Tbx6 gene and the suppression of the expression are carried out depending on the external stimulation. More specifically, cardiac progenitor cells are induced from the expression cassette-introduced pluripotent stem cells by culturing the pluripotent stem cells in the presence of the external stimulation. More preferably, cardiac progenitor cells can be induced from the expression cassette-introduced pluripotent stem cells by culturing the pluripotent stem cells in the presence of the external stimulation and in the absence of serum.

To date, a method of inducing cardiac progenitor cells, comprising culturing ES cells in the presence of a transcriptional factor such as Activin A, BMP4 or Wnt, or a humoral factor, has been disclosed. However, a transcriptional factor for inducing cardiac progenitor cells from pluripotent stem cells and a mechanism thereof have not yet been elucidated. In addition, induction of differentiation into cardiac progenitor cells using a humoral factor has been problematic in that it causes high costs and requires a complicated culture step, and also in that there is induction instability among cell lines.

In contrast, in the method for producing cardiac progenitor cells of the present invention, cardiac progenitor cells, which express the cardiac progenitor cell-specific gene T, Flk-1 or PDGFRα, are induced by inducing a single factor (Tbx6). The percentage of cells expressing these cardiac progenitor cell-specific markers is almost the same as the percentage of the cells obtained by the conventional method of inducing cardiac progenitor cells using Activin A or BMP4. Accordingly, it can be said that the method of the present invention can achieve almost the same induction efficiency more inexpensively and more simply than the conventional method.

Moreover, in the present invention, by suppressing the expression of a Tbx6 gene in pluripotent stem cells after the Tbx6 gene has been expressed in the cells, cardiomyocytes are induced. That is to say, the present invention relates to a method for producing cardiomyocytes from pluripotent stem cells, comprising suppressing the expression of a Tbx6 gene, after the Tbx6 gene has been expressed. The expression of a Tbx6 gene in pluripotent stem cells, into which an expression cassette capable of inducing the expression of the Tbx6 gene in response to external stimulation has been introduced, can be suppressed by culturing the cells in the absence of the external stimulation. Induction of cardiomyocytes from cardiac progenitor cells in the present invention comprises a step of suppressing the expression of a Tbx6 gene. Furthermore, for induction of cardiomyocytes from cardiac progenitor cells, the cells may be cultured in the presence of bFGF or FGF10, as with the conventional method. By performing such a culture, mature beating cardiomyocytes are induced. Further, in the induced cardiomyocytes, the expression of cardiac muscle-specific genes that are α-actinin, cardiac troponin and Nkx2.5 or the formation of a striated structure are confirmed.

That is, the method for producing cardiomyocytes of the present invention comprises suppressing the expression of a Tbx6 gene in pluripotent stem cells, after the Tbx6 gene has been expressed therein. According to the method for producing cardiomyocytes of the present invention, after induction of differentiation into cardiac progenitor cells, if the expression of a Tbx6 gene is suppressed under conditions of the absence of external stimulation, such as suspension of drug administration, functionally mature cardiomyocytes are induced.

### [Pluripotent stem cells]

The pluripotent stem cells used in the present invention may be either embryonic stem (ES) cells or induced pluripotent stem (iPS) cells,

Moreover, the above-described stem cells are not particularly limited, as long as they may be derived from a mamma and they are not derived by destruction of an human embryo. Examples of the mammal include, but are not limited to, a human, a monkey, a horse, a bovine, sheep, a goat, a swine, a dog, a rat, and a mouse. In a certain embodiment the above-described stem cells are derived from a non-human mammal, for example, from a mouse.

ES cells may be stem cells having pluripotency collected from the inner cell mass of an embryo (Strelchenko N et al., (2004) Reprod Biomed Online. 9: 623-629.). ES cells may be established ES cell line. Examples of such an established ES cell line include: a cell line furnished from a cell population obtained by allowing the already established ES cell line to grow; and an ES cell line obtained by thawing a freeze-dried cell line and then culturing it. Such an established ES cell line is available without going through a step of disintegrating a fertilized egg.

ES cells may be established from a single embryonic blastomere at the cleavage stage before the blastocyst stage, without impairing the generating ability of the embryo. Such ES cells can be obtained without destroying a fertilized egg (Klimanskaya I et al., (2006) Nature 444: 481-485; and Chung Y et al., (2008) Cell Stem Cell 2: 113-117).

A method of culturing human-derived ES cells is described, for example, in RIKEN CDB, Human Stem Cell Research Support Office Protocols (2008), Takahashi, K. et al., (Cell (2007), Nov 30: 131, pp. 861-872) and Thomson, J. A. et al. (Science (1998) Nov 6: 282, pp. 1145-1147). A person skilled in the art can appropriately culture mammal-derived ES cells, by applying a known method.

The iPS cells used in the present invention are undifferentiated pluripotent stem cells basically having the same pluripotency as ES cells, which are obtained by introducing several types of reprogramming genes (genes such as Oct3/4, Sox2, c-Myc, Klf4, NANOG, and LIN28) into the somatic cells (fibroblasts, epithelial cells, etc.) of a mammal (including a human) (JP Patent Publication (Kokai) No. 2009-165478 A; Takahashi K et al., (2006) Cell 126: 663-676; Takahashi K et al., (2007) Cell 131: 861-872; etc.). The iPS cells can be cultured by the same method as that applied to ES cells.

### [Tbx6]

In the method for producing cardiac progenitor cells or cardiomyocytes of the present invention, a nucleic acid comprising a nucleotide sequence encoding Tbx6 is introduced into pluripotent stem cells. In the present invention, the amino acid sequence of Tbx6 and the nucleotide sequence encoding the amino acid sequence are known in the present technical field.

A Tbx6 polypeptide (T-box transcriptional factor 6) is a transcriptional factor that binds to T box in the promoter region of a certain gene and recognizes it. The amino acid sequences of Tbx6 polypeptides derived from various species, and nucleotide sequences encoding the Tbx6 polypeptides derived from various species have been known. For example, Genbank Accession Nos. NM_004608.3 (human, nucleotide sequence, SEQ ID NO: 1, CDS 61..1371), NP_004599.2 (human, amino acid sequence, SEQ ID NO: 2), NM_011538.2 (mouse, nucleotide sequence, SEQ ID NO: 3, CDS 25..1335), NP_035668.2 (mouse, amino acid sequence, SEQ ID NO: 4), and the like may be referred to.

Moreover, in one aspect of the present invention, the Tbx6 polypeptide includes a polypeptide, which has an amino acid sequence having a sequence identity of at least 80%, 85%, 90%, 91%, 92%, 93%, 94%, 95%, 96%, 97%, 98%, 99% or 100% to the amino acid sequence shown in SEQ ID NO: 2 or SEQ ID NO: 4, and has a function of inducing pluripotent stem cells to differentiate into cardiac progenitor cells, when it is expressed in the pluripotent stem cells.

Furthermore, in one aspect of the present invention, the Tbx6 polypeptide includes a polypeptide, which has an amino acid sequence comprising a deletion, substitution, insertion or addition of 1 to 50, preferably 1 to 40, more preferably 1 to 20, and further preferably 1 to 10 (for example, 1, 2, 3, 4, 5, 6, 7, 8, 9 or 10) amino acids, or a combination thereof, with respect to the amino acid sequence shown in SEQ ID NO: 2 or SEQ ID NO: 4, and has a function of inducing pluripotent stem cells to differentiate into cardiac progenitor cells, when it is expressed in the pluripotent stem cells.

Further, in one aspect of the present invention, the Tbx6 polypeptide includes a polypeptide, which has an amino acid sequence encoded by a nucleotide sequence having a sequence identity of at least 80%, 85%, 90%, 91%, 92%, 93%, 94%, 95%, 96%, 97%, 98%, 99% or 100% to the nucleotide sequence shown in SEQ ID NO: 1 or SEQ ID NO: 3, and has a function of inducing pluripotent stem cells to differentiate into cardiac progenitor cells, when it is expressed in the pluripotent stem cells.

Still further, in one aspect of the present invention, the Tbx6 gene (nucleic acid) includes a nucleic acid, which has a nucleotide sequence having a sequence identity of at least 80%, 85%, 90%, 91%, 92%, 93%, 94%, 95%, 96%, 97%, 98%, 99% or 100% to the nucleotide sequence shown in SEQ ID NO: 1 or SEQ ID NO: 3, and has a function of inducing pluripotent stem cells to differentiate into cardiac progenitor cells, when the polypeptide encoded by the nucleic acid is expressed in the pluripotent stem cells.

To date, it has been reported that cardiac progenitor cells can be produced from pluripotent stem cells by using a transcriptional factor such as BMP4, Activin A or Wnt, or a humoral factor. In the present invention, however, it is not necessary to culture cells in the presence of such a humoral factor. In addition, in the present invention, in the step of inducing cardiac progenitor cells from pluripotent stem cells, the cells are preferably cultured under serum-free conditions. Moreover, it is not necessary to combine such a transcriptional factor and a humoral factor.

### [Expression of Tbx6 in pluripotent stem cells]

In one aspect of the present invention, a Tbx6 gene can be introduced into pluripotent stem cells *in vitro.* In addition, the pluripotent stem cells are induced to differentiate into cardiac progenitor cells or cardiomyocytes *in vitro.* The induced cardiac progenitor cells or the induced cardiomyocytes can be introduced into an individual body.

In another aspect of the present invention, a Tbx6 gene can be introduced into pluripotent stem cells *in vitro.* In addition, the pluripotent stem cells, into which the Tbx6 gene has been introduced *in vitro,* are introduced into an individual body, and are then induced to differentiate into cardiac progenitor cells or cardiomyocytes *in vivo.*

Introduction of a Tbx6 gene into pluripotent stem cells can be carried out by introducing a nucleic acid comprising a nucleotide sequence encoding Tbx6 into pluripotent stem cells. The thus gene-introduced pluripotent stem cells are induced to differentiate into cardiac progenitor cells, mediated by the expression of Tbx6. Accordingly, the step of introducing a Tbx6 gene into pluripotent stem cells may comprise introducing a Tbx6 polypeptide into pluripotent stem cells. The species as an origin of Tbx6 is preferably identical to the species as an origin of the pluripotent stem cells, such as, for example, a human and a human.

Moreover, the method for producing cardiac progenitor cells of the present invention may comprise a step of transforming pluripotent stem cells with a Tbx6 gene. Furthermore, the method for producing cardiac progenitor cells of the present invention may comprise a step of expressing a Tbx6 gene in pluripotent stem cells. Further, the method for producing cardiomyocytes of the present invention may comprise a step of suppressing the expression of a Tbx6 gene, after the step of expressing the Tbx6 gene in pluripotent stem cells.

In the present invention, the expression of a Tbx6 gene can be stage-specifically controlled by an expression cassette regulating the expression thereof in response to external stimulation. Such an expression cassette is a nucleic acid construct comprising, at least, a promoter capable of inducing the expression of a gene located downstream, in response to external stimulation, and a Tbx6 gene, the expression of which is regulated by the promoter.

The promoter is not particularly limited, as long as it is a promoter capable of inducing the expression of a gene located downstream, in response to external stimulation. In a case where the external stimulation is the presence of a tetracycline-based antibiotic (tetracycline, or a tetracycline derivative such as doxycycline), the promoter is, for example, a promoter capable of inducing the expression of a gene located downstream as a result of the binding of a complex of a tetracycline-based antibiotic and a tetracycline transactivator. On the other hand, in a case where the external stimulation is the absence of a tetracycline-based antibiotic, the promoter is, for example, a promoter capable of inducing the expression of a gene located downstream as a result of dissociation of a tetracycline repressor. Moreover, when the external stimulation is the presence of ecdysteroid (ecdysone, muristerone A, ponasterone A, etc.), the promoter is, for example, a promoter capable of inducing the expression of a gene located downstream as a result of the binding of ecdysteroid with a complex of an ecdysone receptor and a retinoid receptor. Furthermore, in a case where the external stimulation is the presence of FKCsA, the promoter is, for example, a promoter capable of inducing the expression of a gene located downstream as a result of the binding of FKCsA with a complex of a Gal4 DNA-binding domain fused with FKBP12 and a VP16 activator domain fused with cyclophilin.

The expression cassette may comprise an enhancer, a silencer, a selective marker (e.g., a drug resistance gene such as a neomycin resistance gene), an SV40 replication origin and the like, as necessary. In addition, taking into consideration the type of the above-described available promoter, etc., a person skilled in the art can appropriately select an enhancer, a silencer, a selective marker gene, a terminator, etc. from known components, and combine them with one another, so that the skilled person can construct an expression cassette capable of inducing the expression of an HPV-E6/E7 gene at a desired expression level.

Thus, the external stimulation includes a culture in the presence or absence of a drug. For instance, using a tetracycline expression induction system (e.g., TaKaRa, etc.), the expression of a gene of interest is regulated in the presence of doxycycline. For example, a vector is produced, such that a Tbx6 gene can be expressed under the control of a tetracycline-sensitive (Tet-On) promoter. Subsequently, pluripotent stem cells are transfected with the above Tbx6 expression vector to produce transgenic pluripotent stem cells. On the other hand, a Tet-On regulatory plasmid that expresses a reverse tetracycline-controlled transactivator (rtTA) is produced, and the regulatory plasmid is then introduced into the above-described pluripotent stem cells. When doxycycline is added into a medium, rtTA binds to a tetracycline response factor, and the expression of a gene located downstream is induced. When doxycycline is not present in a medium, the expression of a desired gene located downstream is not induced.

The used tetracycline-sensitive expression system may be a commercially available system (e.g., Knockout^{™} Tet RNAi System P (Clontech)), or may be produced by the method described in Dickins RA. et al., (Nature Genetics, 39(7): 914-921 (2007)).

In the present invention; the method of introducing the above-described expression cassette into pluripotent stem cells is not particularly limited, and a known method can be appropriately selected and used. For example, the above-described expression cassette can be inserted into a suitable expression vector, and then, a known transformation method, such as viral infection using a viral vector such as a retroviral vector or an adenoviral vector, a lipofection method, a liposome method, an electroporation method, a calcium phosphate method, a DEAE dextran method, or a microinjection method, can be applied.

Examples of such an expression vector include viral vectors such as lentivirus, retrovirus, herpes virus, adenovirus, adeno-associated virus or Sendai virus, and animal cell expression plasmids. Among others, lentivirus is preferable from the viewpoint that this virus has extremely high efficiency of introduction into the genomic DNA of pluripotent stem cells having relatively low proliferative activity.

Moreover, with regard to the above-described external stimulation, a person skilled in the art can appropriately adjust the additive amount thereof into the after-mentioned medium, while taking into consideration the type of the above-described available promoter, etc. For example, when the external stimulation is the presence of doxycycline, the preferred additive concentration of doxycycline is 0.1 to 10 µg/ml. and more preferably 1 to 2 µg/ml.

Examples of the culture medium, which is used to induce differentiation of the cells into cardiac progenitor cells or cardiomyocytes and to which the above-described external stimulation is added, include IMDM solution, a-MEM solution, and DMEM solution. The culture medium may comprise additives, which are normally added for culture. However, in the case of induction of differentiation of the cells into cardiac progenitor cells, it is not necessary to add serum. In one aspect of the present invention, induction of differentiation into cardiac progenitor cells is carried out in the absence of serum.

In the Tbx6 gene-introduced pluripotent stem cells, doxycycline is allowed to be present for 0 day (e.g., 1, 2, 3, 4, 6, 8, 10, 12, 14, 16, 18, 20, or 22 hours) to 4 days, and preferably for 0 to 3 days. As a result, the pluripotent stem cells are induced to differentiate into cardiac progenitor cells. Induction of the pluripotent stem cells to differentiate into cardiac progenitor cells can be confirmed, for example, by examining the expression of a cardiac progenitor cell-specific marker within a certain period (for example, within 4 days) after initiation of the induction. Moreover, the above-described pluripotent stem cells are induced to differentiate into cardiomyocytes as a result of the subsequent termination of the presence of the external stimulation. Induction of the cells to differentiate into cardiomyocytes can be confirmed, for example, by examining the expression of a cardiomyocyte-specific marker within a certain period (for example, within 3 to 10 days, and preferably within 3 to 5 days) after termination of the external stimulation. For instance, when a Tbx6 gene is introduced into a pluripotent stem cell population, and then, external stimulation that is doxycycline is given to the cell population, it can be confirmed that at least 10%, at least 15%, at least 20%, at least 30%, at least 50%, at least 70%, or at least 90% of the cell population is induced to differentiate into cardiac progenitor cells within 4 days after initiation of the induction, and then, it can also be confirmed that the cells are induced to differentiate into cardiomyocytes within a period of 3 to 10 days, and preferably 3 to 5 days after termination of the external stimulation.

In the method for producing cardiac progenitor cells or cardiomyocytes of the present invention, a certain period of time (for example, 0 to 4 days, and preferably 0 to 3 days, after initiation of the induction) after the step of introducing a Tbx6 gene into pluripotent stem cells a step of sorting a population of the pluripotent stem cells is carried out, so that the ratio of the cardiac progenitor cells can be enriched. Such a sorting step is performed regarding a pluripotent stem cell-specific marker, a pluripotent stem cell-specific protein, a pluripotent stem cell surface antigen, and the like, so that remaining pluripotent stem cells can be removed if they remain. In addition, a sorting step is performed regarding the expression of a marker specific to cardiac progenitor cells or cardiomyocytes, so that the ratio of such cells can be enriched.

Moreover, when the method for producing cardiac progenitor cells or cardiomyocytes of the present invention comprises a step of introducing a nucleic acid comprising a nucleotide sequence encoding a detectable marker into pluripotent stem cells, it can give a means for the sorting step, or a means for confirming induction of differentiation into cardiac progenitor cells or cardiomyocytes. Such a nucleotide sequence encoding a detectable marker is operably linked to a cardiac progenitor cell-specific promoter or a cardiomyocyte-specific promoter, or is linked to a nucleotide sequence encoding a cardiac progenitor cell-specific marker or a nucleotide sequence encoding a cardiomyocyte-specific marker. Examples of the detectable marker include: polypeptides directly generating detectable signals, for example, fluorescent proteins such as GFP, YEP, or BFP; and enzymes generating detectable signals when they act on a substrate, such as luciferase or alkaline phosphatase. Examples of the cardiac progenitor cell-specific promoter include promoters of Mesp1, T, or Flk1 (KDR). Examples of the promoter specific to cardiomyocytes include an α-myosin heavy chain promoter, and a cTnT promoter. The expression of a detectable marker enables detection of cardiac progenitor cells or cardiomyocytes, and as a result, it can give a means for confirming induction of differentiation into cardiac progenitor cells or cardiomyocytes, or sorting the induced cardiac progenitor cells or the induced cardiomyocytes.

In the present description, the phrase "operably linking" is used to mean functional linking between nucleic acids, which gives a desired function such as transcription or translation. For example, this linking includes functional linking between a nucleic acid expression control sequence such as a promoter or a signal sequence, and a second polynucleotide. The expression control sequence has an influence on the transcription and/or translation of the second polynucleotide.

As described above, the nucleic acid comprising the nucleotide sequence encoding the Tbx6 polypeptide can be an expression cassette capable of regulating the expression of Tbx6 in pluripotent stem cells, in response to external stimulation. In another aspect of the present invention, the expression cassette is a viral construct, such as a recombinant adeno-associatcd viral construct (see, for example, U.S. Patent No. 7,078,387), a recombinant adenoviral construct, a recombinant lentiviral construct, etc.

Examples of a suitable expression vector include viral vectors (e.g., vaccinia virus-based viral vectors; polio virus; adenovirus (see, for example, Li et al., Invest Opthalmol Vis Sci 35: 2543 2549, 1994; Borras et al., Gene Ther 6: 515 524, 1999; Li and Davidson, PNAS 92: 7700 7704, 1995; Sakamoto et al., H Gene Ther 5: 1088 1097, 1999; International Publication WO No. 94/12649; International Publication WO No. 93/03769; International Publication WO No. 93/19191; International Publication WO No. 94/28938; International Publication WO No. 95/11984; and International Publication WO No. 95/00655); adeno-associated virus (see, for example, Ali et al., Hum Gene Ther 9: 81 86, 1998, Flannery et al., PNAS 94; 6916 6921, 1997; Bennett et al., Invest Opthalmol Vis Sci 38: 2857-2863, 1997; Jomary et al., Gene Ther4: 683-690, 1997, Rolling et al., Hum Gene Ther 10: 641 648, 1999; Ali et al., Hum Mol Genet 5: 591-594, 1996; Srivastava, International Publication WO No. 93/09239, Samulski et al., J. Vir. (1989) 63: 3822 3828; Mendelson et al., Virol. (1988) 166: 154 165; and Flotte et al., PNAS (1993) 90: 10613-10617); SV40; herpes simplex virus; human immunodeficiency virus (see, for example, Miyoshi et al., PNAS94: 10319-23, 1997; Takahashi et al., J Virol 73: 7812 7816, 1999); retroviral vectors (e.g., vectors derived from murine leukemia virus, spleen necrosis virus, and retrovirus, for example, Rous sarcomere virus, Harvey sarcomere virus, avian leukemia virus, lentivirus, human immunodeficiency virus, myeloproliferative sarcomere virus, and breast cancer virus), but the examples of a suitable expression vector are not limited thereto.

A large number of suitable expression vectors have been known in the present technical field, and many expression vectors are commercially available. The below-mentioned vectors are presented for illustrative purposes, and for eukaryotic host cells, pXT1, pSG5 (Stratagene), pSVK3, pBPV, pMSG, and pSVLSV40 (Pharmacia) are used. However, any other vectors can also be used as long as they are compatible with host cells.

Depending on the used host/vector system, any of many suitable transcriptional and translational regulatory elements, such as constitutive and inducible promoters, transcriptional enhancer elements, and transcriptional terminators, may be used in an expression vector (see, for example, BITTER et al. (1987) METHODS IN ENZYMOLOGY, 153: 516-544).

In another embodiment of the present invention, the Tbx6 CDR sequence may be operably linked to a regulatory element, for example, to a transcriptional regulatory element, such as a promoter. The transcriptional regulatory element functions in eukaryotic cells, such as in mammalian cells. Suitable transcriptional regulatory elements include a promoter and an enhancer. In another embodiment of the present invention, the promoter is an inducible promoter. The expression cassette used in the method for producing cardiac progenitor cells or the method for producing cardiomyocytes of the present invention comprises an inducible promoter.

The inducible promoter comprised in the expression cassette in the present invention induces the expression of a gene operably linked thereto, in response to external stimulation.

In another aspect of the present invention, a nucleotide sequence encoding Tbx6 is operably linked to heart-specific transcriptional regulatory elements (TRE), and in such a case, TRE may include promoters and enhancers. Suitable TRE includes TRE derived from the below-mentioned genes, namely, from myosin light chain-2, α-myosin heavy chain, AE3, cardiac troponin C, and cardiac actin, but the examples of the TRE are not limited thereto (Franz et al. (1997) Cardiovasc. Res. 35: 560-566; Robbins et al. (1995) Ann. N. Y. Acad. Sci. 752: 492-505; Linn et al. (1995) Circ. Res. 76: 584-591;Parmacek et al. (1994) Mol. Cell. Biol. 14: 1870-1885; Hunter et al. (1993) Hypertension 22:608-617; and Sartorelli et al. (1992) Proc. Natl. Acad. Sci. USA 89: 4047-4051.).

Selection of a suitable vector and a suitable promoter is well known in the present technical field. The expression vector may comprise a ribosome binding site for initiation of translation and initiation of transcription. The expression vector may comprise a suitable sequence for amplifying the expression.

Examples of a suitable mammalian expression vector (an expression vector suitable for use in mammalian host cells) include recombinant virus, nucleic acid vectors, such as a plasmid, a bacterial artificial chromosome, a yeast artificial chromosome, a human artificial chromosome, cDNA, cRNA, and a polymerase chain reaction (PCR) product expression cassette, but the examples of a suitable mammalian expression vector are not limited thereto. Examples of a suitable promoter for driving the expression of a nucleotide sequence encoding Tbx6 include inducible promoters, such as those containing a Tet-operator element, but the examples of the suitable promoter are not limited thereto. In some cases, such a mammalian expression vector may encode a marker gene that facilitates discrimination or selection of transfected or infected cells, as well as a Tbx6 polypeptide. Examples of the marker gene include: genes encoding fluorescent proteins such as an enhanced green fluorescent protein, Ds-Red (DsRed: Discosomasp. red fluorescent protein (RFP); Bevis and Glick (2002) Nat. Biotechnol. 20: 83), a yellow fluorescent protein, and a cyan fluorescent protein; and genes encoding proteins that impart resistance to selective agents, such as a neomycin resistance gene, a puromycin resistance gene, and a blasticidin resistance gene, but the examples of the marker gene are not limited thereto.

Examples of a suitable viral vector include: a retrovirus-based viral vector (including lentivirus); adenovirus; and adeno-associated virus, but the examples are not limited thereto. A suitable retrovirus-based vector is Moloney murine leukemia virus (MMLV)-based vector, but other recombinant retroviruses may also be used. Examples of such other recombinant retroviruses include avian leukemia virus, bovine leukemia virus, murine leukemia virus (MLV), mink cell focus inducing virus, murine sarcomere virus, reticuloendotheliosis virus, gibbon ape leukemia virus, Mason Pfizer monkey virus, and Rous sarcomere virus. For instance, U.S. Patent No. 6,333,195 can be referred to.

In another case, the retrovirus-based vector may be a lentivirus-based vector (e.g., human immunodeficiency virus-1 (HIV-1); simian immunodeficiency virus (SIV); or ferine immunodeficiency virus (FIV)), and for instance, Johnston et al. (1999), Journal of Virology, 73(6): 4991-5000 (FIV); Negre D et al. (2002), Current Topics in Microbiology and Immunology, 261:53-74(SIV); and Naldini et al. (1996), Science, 272: 263-267 (HIV) can be referred to.

In order to support incorporation of recombinant retrovirus into target cells, such recombinant retrovirus may comprise a viral polypeptide (e.g., retrovirus env). Such a viral polypeptide has been sufficiently established in the present technical field, and for instance, U.S. Patent No. 5,449,614 can be referred to. The viral polypeptide may be an amphotropic viral polypeptide, for example, amphotropic env. Such an amphotropic viral polypeptide supports incorporation of retrovirus into cells derived from a large number of species including cells that are out of the original host species. The viral polypeptide may be a xenotropic viral polypeptide supporting incorporation of retrovirus into cells that are out of the original host species. In another aspect of the present invention, the viral polypeptide is an ecotropic virus polypeptide, for example, ecotropic env, and such an ecotropic virus polypeptide supports incorporation of retrovirus into the cells of the original host species.

Examples of the viral polypeptide capable of supporting incorporation of retrovirus into cells include MMLV amphotropic env, MMLV ecotropic env, MMLV xenotropic env, vesicular stomatitis virus-g protein (VSV-g), HIV-1 env, gibbon ape leukemia virus (GALV) env, RD114, FeLV-C, FeLV-B, MLV10A1 env gene, and mutants thereof, such as chimera, but the examples are not limited thereto. For instance, Yee et al. (1994), Methods Cell Biol., PtA: 99-112(VSV-G); and U.S. Patent No. 5,449,614 can be referred to. In some cases, in order to promote expression or reinforced binding to a receptor, the viral polypeptide is genetically modified.

In general, recombinant virus is produced by introducing a viral DNA or RNA construct into producer cells. In some cases, the producer cells do not express an exogenous gene. In other cases, the producer cells are "packaging cells" comprising one or more exogenous genes, for example, genes encoding one or more of gag, pol, or env polypeptide, and/or one or more of retrovirus gag, pol, or env polypeptide. Retrovirus packaging cells may comprise a gene encoding a viral polypeptide, for example, VSV-g that supports incorporation of retrovirus into target cells. In some cases, the packaging cells comprise genes encoding one or more lentivirus proteins, such as gag, pol, env, vpr, vpu, vpx, vif, tat, rev, or nef. In some cases, the packaging cells comprise genes encoding adenovirus proteins, such as E1A or E1B, or other adenovirus proteins. For instance, proteins supplied by such packaging cells may be: retrovirus-derived proteins, such as gag, pol, and env; lentivirus-derived proteins, such as gag, pol, env, vpr, vpu, vpx, vif, tat, rev, and nef; and adenovirus-derived proteins, such as E1A and E1B. In many examples, the packaging cells supply proteins derived from viruses that are different from the virus as an origin of the viral vector.

Examples of the packaging cell line include cell lines that can be easily transfected, but the examples are not limited thereto. The packaging cell line can be based on 293T cells, NIH3T3, COS, or HeLa cell lines. The packaging cells are frequently used for packaging a viral vector plasmid comprising a deletion of at least one gene encoding a protein necessary for virus packaging. Cells capable of supplying a deleted protein or polypeptide from a protein encoded by such a viral vector plasmid may be used as packaging cells. Examples of the packaging cell line include Platinum-E (Plat-E); Platinum-A (Plat-A); BOSC23 (ATCCCRL11554); and Bing (ATCC CRL 11270), but are not limited thereto. For instance, Morita et al. (2000), Gene Therapy,7: 1063-1066; Onishi et al. (1996), Experimental Hematology, 24: 324-329; and U.S. Patent No. 6,995,009 can be referred to. Commercially available packaging lines are also useful, and examples of such a commercially available packaging line include Ampho-Pak293 cell line, Eco-Pak2-293 cell line, RetroPackPT67 cell line, and Retro-X Universal Packaging System (all of which are available from Clontech).

The retroviral construct may be derived from a certain range of retroviruses, such as MMLV, HIV-1, SIV or FIV, or from other retroviruses described in the present description. The retroviral construct may encode all viral polypeptides necessary for one or more replication cycles of a specific virus. In some cases, the efficiency of incorporation of virus is improved by addition of other factors or other viral polypeptides. In other cases, as described in U,S. Patent No. 6,872,528, a viral polypeptide encoded by a retroviral construct does not support more than one cycle of replication. Under such circumstances, promotion of virus incorporation can be supported by addition of other factors or other viral polypeptides. In the illustrative embodiment, the recombinant retrovirus is an HIV-1 virus, which comprises a VSV-g polypeptide but does not comprise an HIV-1 env polypeptide.

The retroviral construct may comprise a promoter, a multicloning site, and/or a resistance gene. Examples of the promoter include inducible promoters such as a tetracycline operator element, but the examples are not limited thereto. The retroviral construct may also comprise a packaging signal (e.g., a packaging signal derived from an MFG vector: psi packaging signal). Examples of some retroviral constructs known in the present technical field include pMX, pBabeX, and derivatives thereof, but the examples are not limited thereto. For instance, Onishi et al. (1996), Experimental Hematology, 24: 324-329 can be referred to. In some cases, the retroviral construct is a selfinactivating lentiviral vector (SIN), and for instance, Miyoshi et al. (1998), J. Virol., 72(10): 8150-8157 can be referred to. In some cases, the retroviral construct is LL-CG, LS-CG, CL-CG, CS-CG, CLG, or MFG. Miyoshi et al. (1998), J. Virol., 72(10): 8150-8157; Onishi et al. (1996), Experimental Hematology, 24: 324-329; Riviere et al. (1995), PNAS, 92: 6733-6737 can be referred to. Examples of the viral vector plasmid (or construct) include: retrovirus-based vectors, such as pMXs, pMxs-IB, pMXs-puro, and pMXs-neo (wherein pMXs-IB is a vector that supports a blasticidin resistance gene, instead of a puromycin resistance gene supported by pMXs-puro; Kimatura et al. (2003), Experimental Hematology, 31: 1007-1014; MFG Riviere et al. (1995), Proc. Natl. Acad. Sci. U.S.A., 92: 6733-6737; pBabePuro; Morgenstern et al. (1990), Nucleic Acids Research, 18: 3587-3596; LL-CG, CL-CG, CS-CG, CLG Miyoshi et al. (1998), Journal of Virology, 72: 8150-8157, etc.); and adenovirus-based vectors, such as pAdex1 (Kanegae et al. (1995), Nucleic Acids Research, 23: 3816-3821, etc.). In the illustrative embodiment, the retroviral construct comprises blasticidin (e.g., pMXs-IB), puromycin (e.g., pMXs-puro and pBabePuro); or neomycin (e.g., pMXs-neo). For instance, Morgenstern et al. (1990), Nucleic Acids Research, 8:3587-3596 can be referred to.

Methods for producing recombinant virus from packaging cells and the use thereof have been sufficiently established; and for instance, U.S. Patent Nos. 5,834,256; 6,910,434; 5,591,624; 5,817,491; 7,070,994; and 6,995,009 can be referred to. A majority of methods start with introduction of a viral construct into a packaging cell line. Introduction of such a viral construct includes a calcium phosphate method, a lipofection method (Felgner et al. (1987) Proc. Natl. Acad. Sci. U.S.A. 84: 7413-7417), an electroporation method, a microinjection method, FuGENE Transfection, etc., and any method described in the present description, but is not limited thereto. The viral construct may be introduced into pluripotent stem cells used as hosts by any method known in the present technical field.

A nucleic acid construct may be introduced into host cells by applying various well-known methods, such as non-viral transfection of cells. In the illustrative aspect, the construct is incorporated into a vector, and is then introduced into host cells. Introduction of the construct into cells includes electroporation, calcium phosphate-mediated transition, nucleofection, sonoporation, heat shock, magnetofection, liposome-mediated transition, microinjection, microprojectile-mediated transition (nanoparticles), cationic polymer-mediated transition (DEAE dextran, polyethyleneimine, polyethylene glycol (PEG), etc.), and cell fusion, but is not limited thereto. Introduction of the construct into cells may be carried out by any non-viral transfection known in the present technical field. Other examples of transfection include transfection reagents, such as Lipofectamine, Dojindo Hilymax, Fugene, jetPEI, Effectene, and DreamFect.

### [Pluripotent stem cells comprising Tbx6 gene]

There are also disclosed pluripotent stem cells comprising an exogenous Tbx6 gene, and the pluripotent stem cells are induced to differentiate into cardiac progenitor cells by expressing Tbx6 in the cells. In addition, the pluripotent stem cells comprising an exogenous Tbx6 gene
are induced to differentiate into cardiomyocytes by expressing Tbx6 in the cells, then suppressing the expression of Tbx6, and then culturing the cells in a medium supplemented with bFGF or FGF10. In the present description, the "exogenous" gene means a nucleic acid to be introduced into the cells. There are disclosed pluripotent stem cells comprising an expression cassette capable of inducing the expression of a Tbx6 gene in response to external stimulation. The pluripotent stem cells comprising the expression cassette capable of inducing the expression of a Tbx6 gene in response to external stimulation have an ability by which they are induced to differentiate into cardiac progenitor cells in the presence of the above-described external stimulation. Moreover, the pluripotent stem cells comprising the expression cassette capable of inducing the expression of a Tbx6 gene in response to external stimulation also have an ability by which they are induced to differentiate into cardiomyocytes as a result of cancelling the presence of the external stimulation, following the expression of Tbx6 by the above-described external stimulation (i.e., by converting the presence of the above-described external stimulation to the absence thereof).

It is also disclosed that the pluripotent stem cells comprising an exogenous Tbx6 gene of the present invention may be in an *in-vitro state.* It is also disclosed that the pluripotent stem cells comprising an exogenous Tbx6 gene of the present invention may be mammalian cells, such as human cells, or are derived from human cells.

By screening pluripotent stem cells comprising a Tbx6 gene, a pluripotent stem cell line more stably comprising a Tbx6 gene can be established. The screening method can be appropriately selected and carried out by a person skilled in the art.

### [Cardiac progenitor cells]

In the present invention, the "cardiac progenitor cells" are characterized in that the cells express markers specific to the cardiac progenitor cells. The markers specific to cardiac progenitor cells are factors that are specifically expressed in cardiac progenitor cells (cardiac progenitor cell-related factors). Examples of the marker specific to cardiac progenitor cells include T, Mesp1, Flk1 (KDR), Pdgfrα, and Isl1. Cardiac progenitor cells express at least one, more preferably at least two, and further preferably at least three of such cardiac progenitor cell-specific markers. Cardiac progenitor cells are preferably cells, which express T, Mesp1, and Flk1 (KDR).

In addition, in the present invention, since cardiac progenitor cells are induced from pluripotent stem cells, the cardiac progenitor cells may also be referred to as "induced cardiac progenitor cells."

### [Cardiomyocytes]

In the present invention, the "cardiomyocytes" are characterized in that the cells express markers specific to the cardiomyocytes. The markers specific to cardiomyocytes are factors that are specifically expressed in cardiomyocytes (cardiomyocyte-related factors). Examples of the marker specific to cardiomyocytes include cardiac troponin (cTnT), Nkx2.5, and Actn2. Cardiomyocytes express at least one, more preferably at least two, and further preferably at least three of such cardiomyocyte-specific markers. Cardiomyocytes are preferably cells, which express cTnT and Nkx2.5.

Moreover, in the present invention, the "cardiomyocytes" may be characterized in that the cells beat. Furthermore, in the present invention, the "cardiomyocytes" may also be characterized in that the cells form a striated structure.

Further, in the present invention, since cardiomyocytes are induced from pluripotent stem cells, the cardiomyocytes may also be referred to as "induced cardiomyocytes."

The expression of various markers specific to cardiac progenitor cells or cardiomyocytes can be detected by biochemical or immunochemical approaches (for example, an enzyme-linked immunosorbent assay, an immunohistochemical assay, etc.). Alternatively, the expression of such markers can also be detected by measuring the expression of nucleic acids encoding various markers specific to cardiac progenitor cells or cardiomyocytes. The expression of such nucleic acids encoding various markers specific to cardiac progenitor cells or cardiomyocytes can be confirmed by molecular biological approaches such as RT-PCR or hybridization. Primers or probes used in these approaches can be appropriately designed and produced by a person skilled in the art, using information available from database such as Genbank.

The beating of cardiomyocytes can be confirmed by visual observation or by observing the bright field image thereof. In addition, it is also possible to confirm spontaneous contraction by standard electrophysiological methods such as a patch clamp method.

Moreover, the formation of a striated structure by cardiomyocytes can be confirmed by visual observation or by observing the bright field image thereof. Furthermore, it can also be confirmed by performing immunostaining on proteins contributing to a cardiac muscle structure, such as troponin.

There are also disclosed pluripotent stem cell-derived cardiac progenitor cells (induced cardiac progenitor cells, cardiac progenitor cell-like cells) or pluripotent stem cell-derived cardiomyocytes (induced cardiomyocytes, cardiomyocyte-like cells), which are produced by the above-described method for producing cardiac progenitor cells or cardiomyocytes. Since the induced cardiac progenitor cells or induced cardiomyocytes of the present invention are induced from pluripotent stem cells comprising an exogenous Tbx6 gene, the induced cardiac progenitor cells or induced cardiomyocytes of the present invention also comprise such an exogenous Tbx6 gene. As disclosed herein, the induced cardiac progenitor cells or induced cardiomyocytes of the present invention may be in an *in-vitro* state. It is also disclosed that the induced cardiac progenitor cells or induced cardiomyocytes of the present invention may be mammalian cells such as human cells, or are induced from mammalian cells such as human cells.

As described above, whether or not the cells induced from pluripotent stem cells are cardiac progenitor cells can be confirmed using the expression of a marker specific to cardiac progenitor cells. The cells, in which the expression of a cardiac progenitor cell-specific marker has been confirmed, are also referred to as "cardiac progenitor cell-like cells."

Likewise, as described above, whether or not the cells induced from pluripotent stem cells are cardiomyocytes can be confirmed using the expression of a marker specific to cardiomyocytes. The cells, in which the expression of a cardiac progenitor cell-specific marker has been confirmed, are also referred to as "cardiomyocyte-like cells."

Marker expression can be confirmed at a gene level or at a protein level.

There is also disclosed a composition comprising pluripotent stem cells comprising a Tbx6 gene, or pluripotent stem cell-derived cardiac progenitor cells or pluripotent stem cell-derived cardiomyocytes comprising a Tbx6 gene. The composition as disclosed herein comprises the above-described pluripotent stem cells, or induced cardiac progenitor cells or induced cardiomyocytes, and may further comprise, as suitable components, salts; a buffer; a stabilizer; a protease inhibitor; a cell membrane and/or cell wall preserving compound, such as glycerol or dimethyl sulfoxide; a nutrient medium suitable for cells; and the like.

### [Inducer]

There is also disclosed an inducer for inducing cardiac progenitor cells from pluripotent stem cells, or an inducer for inducing cardiomyocytes from pluripotent stem cells.

It is also disclosed that the inducer of the present invention comprises 1) an expression cassette capable of regulating the expression of a Tbx6 gene in response to external stimulation, which comprises a nucleic acid comprising a nucleotide sequence encoding aTbx6 polypeptide. In addition to the above-described expression cassette, the inducer as disclosed herein may comprise one or more selected from 2) external stimulation, 3) bFGF, FGF10, or the like added to a medium upon induction of cardiomyocytes from cardiac progenitor cells, 4) salts such as NaCl, MgCl, KCl, or MgSO₄; buffers such as a Tris buffer, N-(2-hydroxyethyl)piperazine-N'-2-ethanesulfonic acid (HEPES), 2-(N-morpholino)ethanesulfonic acid (MES), 2-(N-morpholino)ethanesulfonic acid sodium salt (MES), 3-(N-morpholino)propanesulfonic acid (MOPS), or N-tris[hydroxymethyl]methyl-3-aminopropanesulfonic acid (TAPS); solubilizers; detergents including nonionic detergents such as Tween-20; protease inhibitors; glycerol; etc. Moreover, the inducer as disclosed herein may comprise a reagent for introducing a nucleic acid comprising a nucleotide sequence encoding a Tbx6 polypeptide into pluripotent stem cells.

It is also disclosed herein that the inducer as disclosed herein may be directly administerd to an individual body. The inducer as disclosed herein may be useful for inducing pluripotent stem cells to differentiate into cardiac progenitor cells or cardiomyocytes, and this induction can be carried out *in vitro* or *in vivo.* Induction of pluripotent stem cells to differentiate into cardiac progenitor cells or cardiomyocytes can be used to treat various heart failures, and also for researches in the cardiac field.

Accordingly, the inducer as disclosed herein may comprise a pharmaceutically acceptable excipient. Examples of a suitable excipient include water, saline, dextrose, glycerol, ethanol, and a combination thereof. Moreover, as desired, the present inducer may comprise a small amount of auxiliary substance, such as a wettable powder, an emulsifier, or a buffer. Actual methods for preparing such dosage forms have been known. For instance, Remington's Pharmaceutical Sciences. Mack Publishing Company, Easton, Pennsylvania, 17th edition, 1985 can be referred to.

A pharmaceutically acceptable excipient. such as a vehicle, an adjuvant, a carrier or a diluent, can be easily obtained. Further, a pharmaceutically acceptable auxiliary substance, such as a pH adjuster, a buffer, a tension adjuster, a stabilizer or a wettable powder, can be easily purchased.

### [Therapeutic method using cells]

[t is disclosed that the pluripotent stem cells comprising a Tbx6 gene as disclosed herein can be used to treat an individual in need of the treatment. Likewise, the induced cardiac progenitor cells or induced cardiomyocytes as disclosed herein can be used to treat an individual in need of the treatment. The pluripotent stem cells comprising a Tbx6 gene , or the induced cardiac progenitor cells or induced cardiomyocytes as disclosed herein, can be introduced into a recipient individual (an individual in need of treatment), and in such a case, introduction of the pluripotent stem cells comprising a Tbx6 gene of the present invention, or the induced cardiac progenitor cells or induced cardiomyocytes of the present invention, into such a recipient individual provides the treatment of the condition or disorder of the individual. Therefore, there is disclosed a therapeutic method comprising administering the pluripotent stem cells comprising a Tbx6 gene as disclosed herein, or the induced cardiac progenitor cells or induced cardiomyocytes as disclosed herein , to an individual.

For example, it is disclosed that the therapeutic method disclosed herein comprises: i) generating induced cardiac progenitor cells or induced cardiomyocytes *in vitro;* and ii) introducing the induced cardiac progenitor cells or the induced cardiomyocytes into an individual in need thereof.

The therapeutic method as disclosed herein is useful to treat an individual suffering from cardiac or cardiovascular diseases or disorders, such as cardiovascular disease, aneurysm, angina, arrhythmia, atherosclerosis, cerebrovascular accidental disease (stroke), cardiovascular disease, congenital heart disease, congestive heart failure, myocarditis, coronary venous valve disease, scalability artery disease, diastolic dysfunction, endocarditis, hypertension, cardiomyopathy, hypertrophic cardiomyopathy, restrictive cardiomyopathy, coronary disease resulting in ischemic cardiomyopathy, mitral valve prolapse, myocardial infarction (heart attack), or venous thromboembolism.

The unit dosage form of an induced cardiac progenitor cell population or an induced cardiomyocyte population may comprise approximately 10³ to approximately 10⁹ cells, for example, approximately 10³ to approximately 10⁴, approximately 10⁴ to approximately 10⁵, approximately 10⁵ to approximately 10⁶, approximately 10⁶ to approximately 10⁷, approximately 10⁷ to approximately 10⁸, or approximately 10⁸ to approximately 10⁹ cells.

### Examples

### [Example 1] Culture of mouse ES cells

Mouse T-GFP ES cells (obtained from Prof. Gordon Keller, Toronto Medical Discovery Tower MaRS Centre 101 College Street, Room 8-706 Toronto, ON M5G 1L7 CANADA) were inoculated at a concentration of 1.0 × 10⁶ cells/dish into a gelatin-coated 10-cm tissue culture dish (172958, Thermo Scientific), and were then cultured using a medium for ES cells (Table 1) under conditions of 37°C/5% CO₂. Thereafter, subculture was carried out every 2 or 3 days. The T-GFP ES cells are cells that exhibit positive to GFP, when T as a marker transcriptional factor for cardiac mesoderm is expressed.

### [Table 1]

**(Table 1) Medium for ES cells**

| | |
|---|---|
| FBS (Fetal Bovine Serum) (Thermo Scientific, SH30071.03) | 50 mL |
| Glasgow Minimum Essential Medium (Sigma, G5154) | 500 mL |
| PSA | 5 mL |
| Sodium Pyruvate (Sigma, S8636) | 5 mL |
| GlutaMAX (lnvitrogen, 35050-061) | 5 mL |
| Non-essential amino acids solution 100x (Invitrogen, M7145) | 5 mL |
| Mercaptoethanol (Sigma, 21985) | 0.5 mL |
| Leukemia inhibitory factor (Millipore, ESG1106) | 0.1 mL |
| CHIR99021 (Selleckchem, S1263) | 0.15 mL |
| PD0325901 (Selleckchem, S1036) | 0.05 mL |

### [Example 2] Induction of cardiac progenitor cells and cardiomyocytes from mouse ES cells, using cytokine.

The ES cells, which had been subcultured in Example 1, were washed with PBS (-) after aspiration of the medium for ES cells, and thereafter, 2 mL of 0.05% Trypsin-EDTA was added to each dish, and the cells was then left at rest under conditions of 37°C/5% CO₂ for 3 minutes. After the floating of the cells in the culture medium had been confirmed, the reaction was neutralized with a solution consisting of 1 mL of FBS/7 mL of IMDM, and were then recovered in a 15-mL tube (430791, Corning). The recovered cells were centrifuged under conditions of 100 RPM for 3 minutes. Thereafter, a supernatant was aspirated, 10 mL of IMDM was then added to a cell precipitate, and the number of cells was then counted. A solution of 7.5 × 10⁵ cells was transferred into a new 15-mL tube, and was then centrifuged under conditions of 1100 RPM for 3 minutes, followed by aspiration of the supernatant. A serum-free differentiation medium (Table 3) prepared by adding L-ascorbic acid and 1-thioglycerol to 10 mL of a serum-free medium (Table 2) was added to the cell precipitate, and the obtained cells were then inoculated into a 10-cm sterilized petri dish (SH90-15, IWAKI) (Day 0).

### [Table 2]

**(Table 2) Serum-free medium**

| | |
|---|---|
| Iscove's modified Dulbecco's medium (IMDM) (Invitrogen, 12440-053) | 750 mL |
| Ham-F12 medium (Wako, 087-08335) | 250 mL |
| N2 supplements (lnvitrogen, 17502-048) | 5 mL |
| B27 supplements (Invitrogen, 17504-044) | 10 mL |
| 7.5% bovine serum albumin (Invitrogen, P2489) | 6.6 mL |
| PSA | 7.5 mL |
| GlutaMAX (Invitrogen, 35050-061) | 7.5 mL |

### [Table 3]

**(Table 3) Serum-free differentiation medium**

| | |
|---|---|
| Serum-free medium (Table 2) | 10 mL |
| 5 mg/ml L-ascorbic acid (Sigma. A4544) | 0.1 mL |
| 13 µl of 1-thioglycerol (Sigma, M6145)/1 ml of IMDM solution | 0.03 mL |

On Day 1, the petri dish was stirred in all directions, so that the cells were uniformly distributed.

On Day 2, the formed embryoid body, together with the medium, was transferred into a 15-mL tube, and was then centrifuged under conditions of 700 RPM for 3 minutes, followed by aspiration of the supernatant. Thereafter, 2 mL of 0.05% Trypsin-EDTA was added to the precipitated embryoid body, and the tube was then left at rest under conditions of 37°C/5% CO₂ for 2 minutes. After completion of repeated pipetting, the reaction was neutralized with a solution consisting of 1 mL of FBS/7 mL of IMDM, and was then centrifuged under conditions of 1200 RPM for 3 minutes, followed by aspiration of the supernatant. The obtained precipitate was diluted with 10 mL of IMDM, and the number of cells was then counted. A solution of 7.5 × 10⁵ cells was transferred into a new 15-mL tube, and was then centrifuged under conditions of 1100 RPM for 3 minutes, followed by aspiration of the supernatant. 10 mL of a medium for use in induction of differentiation into cardiac progenitor cells (Table 4) was added to the cell precipitate, and the cells were then inoculated into the sterilized petri dish.

### [Table 4]

**(Table 4) Medium for use in induction of differentiation into cardiac progenitor cells**

| | |
|---|---|
| Serum-free differentiation medium (Table 3) | 10 mL |
| 10 ng/µl Recombinant human Activin A (R&D Systems, 338-AC) | 5 µL |
| 10 ng/µl Recombinant human BMP4 (R&D Systems, 314-BP) | 0.7 µL |
| 5 ng/µl Recombinant human VEGF (R&D Systems, 293-VE) | 10 µL |

On Day 3, the petri dish was stirred in all directions, so that the cells were uniformly distributed.

On Day 4, cardiac progenitor cells were induced. As in the case of Day 2, the formed embryoid body, together with the medium, was transferred into a 15-mL tube, and was then centrifuged under conditions of 700 RPM for 3 minutes, followed by aspiration of the supernatant. Thereafter, 2 mL of 0.05% Trypsin-EDTA was added to the precipitated embryoid body, and the tube was then left at rest under conditions of 37°C/5% CO₂ for 2 minutes. After completion of repeated pipetting, the reaction was neutralized with a solution consisting of 1 mL of FBS/7 mL of IMDM, and was then centrifuged under conditions of 1200 RPM for 3 minutes, followed by aspiration of the supernatant. The obtained precipitate was diluted with 10 mL of IMDM, and the number of cells was then counted. A solution of 6.0 × 10⁵ cells/well was transferred into a new 15-mL tube, and was then centrifuged under conditions of 1100 RPM for 3 minutes, followed by aspiration of the supernatant. A medium for use in induction of cardiomyocytes (Table 5) was added to the cell precipitate, and the obtained cells were then inoculated into a gelatin-coated 24 Well Cell Culture Multiwell Plate (353047, FALCON). The cells were left at rest for 15 minutes for adhesion of the cells, and were then cultured under conditions of 37°C/5% CO₂. Thereafter, the medium was exchanged with a new medium every 2 or 3 days. When favorable induction was obtained, myocardial beating was observed from Day 7.

### [Table 5]

**(Table 5 Medium for use in induction of cardiomyocytes**

| | |
|---|---|
| StemPro(R) 34 SFM (Invitrogen, 10639-011) with Nutrient Supplement | 10 mL |
| GlutaMAX (Invitrogen, 35050-061) | 100 µL |
| 5mg/mL Ascrobic Acid (Sigma, A-4544) | 200 µL |
| 5 ng/µL Recombinant Human VEGF165 (R & D Systems, 293-VE-050) | 10 µL |
| 10 ng/µL Recombinant Human FGF basic 146 aa (R & D Systems, 233-FB-025) | 10 µL |
| 50 ng/µL Recombinant Human FGF10 (R & D Systems, 345-FG-025) | 5 µL |

### [Example 3] Introduction of tetracycline-regulated gene expression system into mouse ES cells

In Example 3, a Tbx6 gene was introduced into mouse ES cells, using lentivirus. The used vector is able to stage-specifically control the expression of the introduced gene by addition of doxycycline (Figure 1).

293T cells were inoculated at a concentration of 6.0 × 10⁶ cells/dish into a 10-cm tissue culture dish (353047, Corning), and were then left at rest under conditions of 37°C/5% CO₂ (Day 1).

On the following day (Day 2), Solution A, in which 36 µL of Lipofectamine (1168-019, Invitrogen) was mixed with 1500 µL of Opti-MEM (31985-070, Gibco), and Solution B, in which 25 µg of CSIV-TRE-RfA-UbC-KT-Tbx6 plasmid, 10 µg of pMDL, and 10 µg of pCMV-VSV-G-RSV-Rev were mixed with 1500 µL of Opti-MEM, were prepared, and were then left at rest at room temperature for 5 minutes. Thereafter, Solution C was prepared by mixing the Solution A with Solution B, and was then left at rest for 20 minutes. After the medium had been aspirated from the 293T cells prepared on Day 1, and a solution prepared by mixing 3 mL of the Solution C with 7 mL of a medium for 293T cells (Table 6-1) was then added to the 293T cells. The obtained cells were cultured under conditions of 37°C/5% CO₂ (transfection).

### [Table 6-1]

**(Table 6-1) Medium for 293T cells**

| | |
|---|---|
| FBS (Fetal Bovine Serum) (Thermo Scientific, SH30071.03) | 50 mL |
| DMEM (WAKO, 044-29765) | 450 mL |

Twenty-four hours later (Day 3), the medium was exchanged with a medium prepared by mixing 10 µM Forskolin (66575-29-9, WAKO) into a culture medium for 293T cells.

On the other hand, for virus solution infection on Day 4, ES cells were inoculated at a concentration of 3.0 × 10⁵ cells/well into a gelatin-coated 12 Well Cell Culture Multiwell Plate (353043, FALCON), and were then cultured in a medium for ES cells.

Forty-eight hours after the transfection (Day 4), the culture supernatant was filtrated through a Minisart filter with a pore size of 0.45 µm (17598, Sartorius Stedim Biotech), and was then recovered into a 50-mL tube (430829, Corning). The recovered supernatant (10 mL) was ultracentrifuged using an ultra-high speed centrifuge (Optima XL, Beckman) at 23000 RPM for 2 hours. After completion of the centrifugation, the supernatant was aspirated, and the precipitate was then dissolved in 1 mL of a medium for ES cells, comprising 1.3 µL of Polybrene Transfection Reagent (10 mg/mL) (#TR-1003-G, Millipore). The obtained solution was defined as a lentivirus solution with respect to Tbx6. The medium for ES cells, which had been cultured from the previous day (Day 3), was exchanged with the lentivirus solution, so that the cells were infected with the virus (infection).

On Day 5, the lentivirus solution was exchanged with a medium for ES cells, and the culture was further continued. After the infected cells had become confluent, the cells were subcultured in a 10-cm tissue culture dish. At a stage at which the colony had an appropriate size, the colony was collected and separated using a tip of Pipetman under a microscope, and ES cells were then cloned from a single colony.

### [Example 4] Induction of cardiac progenitor cells by Tbx6 under serum-free conditions

On Day 0, the ES cells cloned in Example 3 were inoculated by the same method as that applied in Example 2. At that time, 2 µg/mL doxycycline (D9891, Sigma) was added to a medium for serum differentiation.

On Day 1, a petri dish was stirred in the same manner as that of Example 2.

On Day 2, the cells were inoculated into a sterilized petri dish in the same manner as that of Example 2. However, the medium used herein was a doxycycline-added medium for serum differentiation, as with the medium used on Day 0.

On Day 3, the petri dish was stirred in the same manner as that of Example 2.

On Day 4, the induced cardiac progenitor cells were analyzed. In addition, after Day 4, the experiment was carried out in the same manner as that of Example 3.

Doxycycline was allowed to be present for 3 days after initiation of the induction (Figure 3, "Dox On"), and Tbx6 was allowed to express for 3 days. Figure 2 shows the results obtained by measuring the expression level of Tb×6 mRNA in a cell line obtained by cloning. The expression of Tb×6 mRNA was observed in the presence (+) of doxycycline (Dox).

### [Example 5] Verification of serum-free induction of differentiation into cardiac progenitor cells and cardiomyocytes by Tbx6

### [FACS analysis method]

An embryoid body comprising cardiac progenitor cells, together with the medium, was recovered, and 0.25% Trypsin-EDTA was then added to the embryoid body. The obtained embryoid body was left at rest under conditions of 37°C/5% CO₂ for 2 minutes, so as to obtain single cells. After neutralization of Trypsin-EDTA, the cells were recovered in a 15-mL tube (430791, Corning). The recovered cells were centrifuged under conditions of 1500 rpm/5 minutes/4°C. After aspiration of the supernatant, 100 µL of a solution for performing FACS (5% FBS/PBS) (Table 6-2) was added to the cell precipitate, and the cells were fully suspended.

### [Table 6-2]

**(Table 6-2) Solution for performing FACS**

| | |
|---|---|
| FBS (Fetal Bovine Serum) (BioWest) | 10 mL |
| PBS (-) (WAKO, 045-29795) | 190 mL |

This suspension was reacted for 30 minutes with anti-mouse Flk1 conjugated with APC antibody (17-5821, Ebioscience,) and with anti-mouse PdgfR-a (CD140a) conjugated with BV421 antibody (562774, BD Biosciences), at a concentration of 50 : 1, respectively. After completion of centrifugation, the antibody was removed, and 350 µL of a solution for performing FACS was then added again to the precipitated cell mass. The obtained suspension was filtered with a 5-mL polystyrene round tube with cell strainer cap (REF 353335m FALCON) to obtain a sample for FACS.

Using flow cytometry (FACS ArialIIu, Nippon Becton Dickinson Company, Ltd.), the above-described sample was measured.

### [Verification of induction of cardiac progenitor cells by immunostaining]

Figure 4 includes GFP images obtained by immunostaining. The ES cells used in the Examples (T-GFP ES cells) are cells that exhibit positive to GFP, when T as a marker transcriptional factor for cardiac mesoderm is expressed. The term "Off' indicates the results of the cell line of Example 4, to which doxycycline is not added; the term "On" indicates the results of the cell line of Example 4, in which Tbx6 has been induced by addition of doxycycline; and the term "BMP4/Activin" indicates the results of cells (Example 2) differentiated from ES cells with the use of BMP4 and Activin (Day 4). In the case of mouse ES cells (Examples 3 and 4), into which a Tbx6 gene had been introduced using a tetracycline-regulated gene expression system, the cells were exhibited positive to GFP by addition of doxycycline. Hence, it is found that T as a marker transcriptional factor for cardiac mesoderm was expressed. Moreover, in the ES cells of Examples 3 and 4, GFP was expressed by doxycycline at the same level as that in the cells (Example 2) to which cytokines such as BMP4 and Activin were used.

Therefore, it was demonstrated that cardiac progenitor cells can be induced from ES cells under serum-free conditions and without using humoral factors, by introducing a Tbx6 gene into the ES cells. In addition, in the present Example, it was also demonstrated that cardiac progenitor cells can be induced at the same induction efficiency as that in the case of using cytokines (BMP4/Activin).

### [Verification of induction of differentiation into cardiac progenitor cells by FACS]

Figure 5 shows the results obtained by measuring the expression of GFP by FACS. As described above, the ES cells used in the Examples exhibit positive to GFP, when T as a marker transcriptional factor for cardiac mesoderm is expressed. The term "Off" indicates the results of the cell line of Example 4, to which doxycycline is not added; the term "On" indicates the results of the cell line of Example 4, in which Tbx6 has been induced by addition of doxycycline; and the term "BMP4/Activin" indicates the results of cells (Example 2) differentiated from ES cells with the use of BMP4 and Activin (Day 4). In the case of mouse ES cells (Examples 3 and 4), into which a Tbx6 gene had been introduced using a tetracycline-regulated gene expression system, the percentage of the cells exhibited positive to GFP expression as a result of addition of doxycycline was 81.3%, whereas the percentage of the GFP-positive cells was 95.6% in the case of addition of "BMP4/Activin." From these results, it was found that when a Tbx6 gene is introduced into ES cells so that Tbx6 is expressed therein, T as a marker transcriptional factor for cardiac mesoderm is expressed, and cardiac progenitor cells are induced.

It has been known that cardiac progenitor cells become positive to two surface markers, Flk-1 and PDGFRα. The results obtained by measuring the expression of Flk-1 and PDGFRα are shown in Figure 6 (Day 4). When a Tbx6 gene was introduced into ES cells so that Tbx6 was expressed therein (Figure 6, "on"), the percentage of Flk-1 (+)/PDGFRα (+) cells was 37.5%. On the other hand, in the case of "BMP4/Activin," the percentage of Flk-1 (+)/PDGFRα (+) cells was 35.4%. From these results, it was found that cardiac progenitor cells are induced from mouse ES cells, into which a Tbx6 gene has been introduced using a tetracycline-regulated gene expression system and has been expressed therein. In addition, it was also found that, according to the present invention, cardiac progenitor cells can be induced with the same level of induction efficiency as in the case of inducing the cells by a conventional method using cytokines such as BMP4 and Activin.

Therefore, from these Examples, it was elucidated that cardiac progenitor cells are induced from Es cells only by the expression of Tb×6. Moreover, it was also elucidated that the method of the present invention is able to induce cardiac progenitor cells with the same induction efficiency as a conventional method using serum, humoral factors and the like.

### [Verification of induction of cardiomyocytes]

After Day 3 of induction of differentiation into cardiac progenitor cells, the expression of Tb×6 was suspended, so that mature differentiated beating cardiomyocytes were induced. Specifically, doxycycline was allowed to be present in the medium only for 3 days after initiation of the induction (Figure 3), and thereafter, induction of cardiomyocytes was examined on Day 14.

First, the expression of α-actinin (α-Actinin) and cardiac troponin T (cTnT) serving as myocardial markers was measured by immunostaining. As a result, in the cells that had been treated with doxycycline (Figure 7, "Dox On"), the expression of structural proteins such as troponinT and actinin was confirmed. In contrast, in the cells that had not been treated with doxycycline (Figure 7, "Dox Off"), the expression of these proteins was not detected.

The results obtained by measuring the expression of troponinT by FACS are shown in Figure 8. In Tbx6 gene-induced cells which had been treated with doxycycline for 3 days (Figure 8, "On"), the percentage of the cells, in which the expression of troponin T was confirmed, was 66.7%. On the other hand, in ES cells induced with conventional humoral factors such as BMP4 and Activin, the percentage of the cells, in which the expression of troponin T was confirmed, was 63.9%. Accordingly, it was found that the method of the present invention using Tbx6 has the efficiency of inducing troponinT that is equivalent to that of a conventional method using humoral factors.

In addition, the expression of the mRNA of cardiac muscle-related markers was examined (Figure 9). Actinin 2 (Actn2), troponin T2 (TnnT2), Nkx2.5, and the like are markers expressing specifically to cardiomyocytes. As shown in Figure 9, in Tbx6 gene-induced cells, which had been treated with doxycycline for 3 days (Figure 9, "On"), the mRNA expression of these marker genes was confirmed. The mRNA expression of these marker genes was at the same level as that in cardiomyocytes induced from ES cells according to a conventional method (BMP4/Activin).

Moreover, it was also confirmed that cardiomyocytes differentiated from Tbx6-induced cardiac progenitor cells were beating.

As such, ES cells comprising a Tbx6 gene are first induced to differentiate into cardiac progenitor cells by the expression of the Tbx6 gene, and then, the expression of Tbx6 is controlled, so that the cardiac progenitor cells are induced to differentiate into cardiomyocytes, as in the case of using BMP4/Activin. Accordingly, it was elucidated that cardiac progenitor cells are induced from ES cells by expressing Tbx6 in the ES cells, and that the cardiac progenitor cells are then induced to differentiate into cardiomyocytes by regulating the expression of Tbx6.

### [Example 6] Culture of human iPS cells

Human iPS cells (obtained from Dept. of Fundamental Cell Technology, Center for iPS Cell Research and Application (CiRA), Kyoto University) were inoculated at a concentration of about 10 cells/colony into a 10-cm tissue culture dish coated with hES-qualified Matrix (354277, Corning), and were then cultured using a medium for human iPS cells (mTESRTM^{™}1 (05850, STEMCELL)) under conditions of 37°C/5% CO₂. Thereafter, subculture was carried out every 7 days.

### [Example 7] Induction of cardiac progenitor cells and cardiomyocytes from human iPS cells, using cytokines

Four days before initiation of the induction (Day -4), a medium for human iPS cells was aspirated, and human iPS cells were washed with PBS (-). Then, 500 µL of StemPro Accutase Cell dissociation Reagent (A1115-01, GIBCO) was added to each dish, and was then left at rest under conditions of 37°C/5% CO₂ for 3 minutes. Thereafter, the resulting cells were washed with PBS (-), and were then suspended in a medium for human iPS cells. The suspension was passed through a 40-µm filter to recover single cells. The number of cells was counted, and the cells were then inoculated at a concentration of 1 × 10⁵ cells/well into a 12 Well Cell Culture Multiwell Plate coated with Matrigel.

On Day 0, after the cells had been washed with PBS, the medium was exchanged with a medium for induction of human cardiac progenitor cells (Table 7), and the cells were then cultured for 2 days. On Day 2, the cells were washed with PBS (-), and the medium was then exchanged with a medium for human serum-free differentiation (Table 8). On Day 3, the medium was exchanged with a medium for induction of human cardiomyocytes, and the cells were then cultured for 4 days (Table 9).

### [Table 7]

**(Table 7) Medium for induction of human cardiac progenitor cells**

| | |
|---|---|
| StemPro(R) 34 SFM (Invitrogen, 10639-011) with Nutrient Supplement | 10 mL |
| GlutaMAX (Invitrogen, 35050-061) | 100 µL |
| 10 ng/µL Recombinant human BMP4 (R&D Systems, 314-BP) | 5 µL |
| 10 ng/µL Recombinant human Activin A (R&D Systems, 338-AC) | 12.5 µL |
| 10 ng/µL Recombinant Human FGF basic (Wako, 064-04541) | 10 µL |

### [Table 8]

**(Table 8) Medium for human serum-free differentiation**

| | |
|---|---|
| StemPro(R) 34 SFM (Invitrogen, 10639-011) with Nutrient Supplement | 10 mL |
| GlutaMAX (Invitrogen, 35050-061) | 100 µL |

### [Table 9]

**(Table 9) Medium for induction of human cardiomyocytes**

| | |
|---|---|
| StemPro(R) 34 SFM (Invitrogen, 10639-011) with Nutrient Supplement | 10 mL |
| GlutaMAX (Invitrogen, 35050-061) | 100 µL |
| 10 mM IWR-1 (SIGMA, 10161) | 5 µL |
| 5 ng/µL Recombinant Human VEGF (AtGen, ATGP1449) | 10 µL |

After Day 7, the cells were cultured in a medium prepared by adding VEGF to the medium for human serum-free differentiation, and the medium was then exchanged with a fresh medium every 2 or 3 days. When favorable induction was obtained, myocardial beating was observed from Day 8.

### [Example 8] Introduction of tetracycline-regulated gene expression system into human iPS cells

A lentivirus solution was prepared in the same manner as that of Example 3 (mouse ES cells).

A 6 Well Cell Culture Multiwell Plate had previously been coated with Matrigel, and 2 × 10⁴ human iPS cells were added to 2 mL of the virus solution. The obtained cells were inoculated into the coated plate.

From the following day, the cells were cultured using mTESR1. After the infected cells had become confluent, they were subcultured in a 10-cm tissue culture dish. At a stage at which the colony had an appropriate size, the colony was collected and separated using a tip of Pipetman under a microscope, and human iPS cells were then cloned from a single colony. As a result, a human iPS cell line, which stage-specifically expresses Tbx6, was established.

### [Example 9] Induction of human cardiac progenitor cells and cardiomyocytes by Tbx6 under serum-free conditions

Four days before initiation of the induction (Day -4), human iPS cells, which had been cloned in Example 8, were inoculated by the same method as that applied in Example 7 (Figure 10, protocols).

One day before initiation of the induction (Day -1), 2 µg/mL doxycycline was added to mTESR1.

On Day 0, the cells were washed with PBS (-), and the medium was then exchanged with a medium for human serum-free differentiation, followed by performing culture for 3 days.

After Day 3, the cells were cultured by the same method as that applied in Example 7.

### [Verification of induction of cardiac progenitor cells]

Induction of cardiac progenitor cells from iPS cells was verified, as in the case of using ES cells. Differing from the ES cells, in the case of using human iPS cells, Tbx6 was allowed to express in the human iPS cells only for 1 day before initiation of the induction (Day -1) (Figure 10, protocols, Dox On).

When Tbx6 was allowed to express in Tbx6 gene-introduced human iPS cells by treating the cells with doxycycline, the expression of the cardiac mesoderm marker transcriptional factor T was confirmed on Day 1 by FACS and immunostaining (Figures 10A and 10B).

Therefore, it was elucidated that human cardiac progenitor cells are induced from human iPS cells by the expression of Tb×6.

### [Verification of induction of cardiomyocytes]

When Tbx6 was allowed to express in Tbx6 gene-introduced human iPS cells by treating the cells with doxycycline, the expression of troponin T as a cardiomyocyte marker was confirmed on Day 14 by FACS (Figure 10C). Moreover, it was also confirmed that the cardiac progenitor cells induced by the expression of Tbx6 differentiate into mature beating cardiomyocytes (Day 14).

Furthermore, in addition to the expression of troponin T, the expression of a marker for cardiac progenitor cell-derived vascular endothelial cells (CD31) and a marker for cardiac progenitor cell-derived smooth muscle (Calponin) was observed (Figure 10D).

Accordingly, it was demonstrated that cardiac progenitor cells induced from human iPS cells by Tbx6 can differentiate, not only into human cardiac muscle, but also into vascular endothelial cells or smooth muscle cells. Therefore, it was demonstrated that cardiac progenitor cells induced from human iPS cells by Tbx6 have pluripotency.

### Industrial Applicability

According to the present invention, a method of inducing cardiac progenitor cells and cardiomyocytes from pluripotent stem cells is provided. In addition, pluripotent stem cells, which are induced to differentiate into cardiac progenitor cells and/or cardiomyocytes, are disclosed. The pluripotent stem cells of the present invention, which are induced to differentiate into the cardiac muscle, are able to control the induction of differentiation into cardiac progenitor cells and the induction of differentiation into cardiomyocytes by turning the expression of Tbx6 on and off.

More specifically, according to the present invention, using a system capable of regulating gene expression by external stimulation such as administration of doxycycline, cardiac progenitor cells are induced from mouse ES cells or human iPS cells, and further, by removing the external stimulation, the expression of Tb×6 is suppressed, so that functionally mature cardiomyocytes can be induced.

The method for producing cardiac progenitor cells and cardiomyocytes of the present invention has the efficiency of inducing cardiac progenitor cells and cardiomyocytes that is 2 to 3 times higher than that of Eomes, which has been previously reported regarding mouse cells. Accordingly, cardiac progenitor cells and cardiomyocytes can be produced with high efficiency according to the method for producing cardiac progenitor cells and cardiomyocytes of the present invention.

### Sequence Listing Free Text

SEQ ID NO: 1: Nucleotide sequence of human Tbx6.
SEQ ID NO: 2: Amino acid sequence of human Tbx6.
SEQ ID NO: 3: Nucleotide sequence of mouse Tbx6.
SEQ ID NO: 4: Amino acid sequence of mouse Tbx6.

## Claims

1. A method for producing cardiac progenitor cells from pluripotent stem cells, comprising expressing a Tbx6 gene in the pluripotent stem cells.

2. A method for producing cardiomyocytes from pluripotent stem cells, comprising:
(i) a step of inducing cardiac progenitor cells from pluripotent stem cells, comprising expressing a Tbx6 gene in the pluripotent stem cells, and
(ii) a step of inducing cardiomyocytes from the cardiac progenitor cells induced in the step (i), comprising suppressing the expression of the Tbx6 gene.

3. The method according to claim 1 or 2, wherein the expression of the Tbx6 gene and the suppression of the expression are carried out using an expression cassette that regulates the expression in response to external stimulation.

4. The method according to any one of claims 1 to 3, wherein the step of expressing the Tbx6 gene in the pluripotent stem cells comprises:
a step of introducing an expression cassette capable of inducing the expression of the Tbx6 gene in response to external stimulation, into the pluripotent stem cells; and
a step of culturing the pluripotent stem cells, into which the expression cassette has been introduced, in the presence of external stimulation.

5. The method according to any one of claims 1 to 4, wherein the step of expressing the Tbx6 gene in the pluripotent stem cells comprises:
a step of introducing an expression cassette capable of inducing the expression of the Tbx6 gene in response to external stimulation, into the pluripotent stem cells; and
a step of culturing the pluripotent stem cells, into which the expression cassette has been introduced, in the presence of external stimulation and in the absence of serum.

6. The method according to any one of claims 2 to 5, wherein the step of suppressing the expression of the Tbx6 gene comprises:
a step of culturing the pluripotent stem cells, into which the expression cassette capable of inducing the expression of the Tbx6 gene in response to external stimulation has been introduced, in the absence of external stimulation.

7. The method according to any one of claims 1 to 6, wherein the pluripotent stem cells are ES cells or iPS cells.

8. The method according to any one of claims 1 to 7, wherein the external factor is tetracycline or doxycycline.

## Patentansprüche

1. Verfahren zur Herstellung kardialer Vorläuferzellen aus pluripotenten Stammzellen, umfassend das Exprimieren eines Tbx6-Gens in den pluripotenten Stammzellen.

2. Verfahren zum Herstellen von Kardiomyozyten aus pluripotenten Stammzellen, umfassend:
(i) einen Schritt des Induzierens kardialer Vorläuferzellen aus pluripotenten Stammzellen, umfassend das Exprimieren eines Tbx6-Gens in den pluripotenten Stammzellen, und
(ii) einen Schritt des Induzierens von Kardiomyozyten aus den kardialen Vorläuferzellen, die in Schritt (i) induziert wurden, umfassend das Unterdrücken der Expression des Tbx6-Gens.

3. Verfahren nach Anspruch 1 oder 2, wobei die Expression des Tbx6-Gens und die Unterdrückung der Expression unter Verwendung einer Expressionskassette durchgeführt werden, die die Expression als Antwort auf externe Stimulation reguliert.

4. Verfahren nach einem der Ansprüche 1 bis 3, wobei der Schritt des Exprimierens des Tbx6-Gens in den pluripotenten Stammzellen umfasst:
einen Schritt des Einbringens einer Expressionskassette in die pluripotenten Stammzellen, die in der Lage ist, die Expression des Tbx6-Gens als Antwort auf externe Stimulation zu induzieren; und
einen Schritt des Züchtens der pluripotenten Stammzellen, in die die Expressionskassette eingebracht wurde, in Anwesenheit externer Stimulation.

5. Verfahren nach einem der Ansprüche 1 bis 4, wobei der Schritt des Exprimierens des Tbx6-Gens in die pluripotenten Stammzellen umfasst:
einen Schritt des Einbringens einer Expressionskassette in die pluripotenten Stammzellen, die in der Lage ist, die Expression des Tbx6-Gens als Antwort auf externe Stimulation zu induzieren; und
einen Schritt des Züchtens der pluripotenten Stammzellen, in die die Expressionskassette eingebracht wurde, in Anwesenheit externer Stimulation und in Abwesenheit von Serum.

6. Verfahren nach einem der Ansprüche 2 bis 5, wobei der Schritt des Unterdrückens der Expression des Tbx6-Gens umfasst:
einen Schritt des Züchtens der pluripotenten Stammzellen, in die die Expressionskassette, die in der Lage ist, die Expression des Tbx6-Gens als Antwort auf externe Stimulation zu induzieren, eingebracht wurde, ohne externe Stimulation.

7. Verfahren nach einem der Ansprüche 1 bis 6, wobei die pluripotenten Stammzellen ES-Zellen oder iPS-Zellen sind.

8. Verfahren nach einem der Ansprüche 1 bis 7, wobei der externe Faktor Tetracyclin oder Doxycyclin ist.

## Revendications

1. Méthode pour produire des cellules progénitrices cardiaques à partir de cellules souches pluripotentes, comprenant l'expression d'un gène de Tbx6 dans les cellules souches pluripotentes.

2. Méthode pour produire des cardiomyocytes à partir de cellules souches pluripotentes, comprenant :
(i) une étape d'induction de cellules progénitrices cardiaques à partir de cellules souches pluripotentes, comprenant l'expression d'un gène de Tbx6 dans les cellules souches pluripotentes, et
(ii) une étape d'induction de cardiomyocytes à partir des cellules progénitrices cardiaques induites dans l'étape (i), comprenant la suppression de l'expression du gène de Tbx6.

3. Méthode selon la revendication 1 ou 2, dans laquelle l'expression du gène de Tbx6 et la suppression de l'expression sont effectuées par utilisation d'une cassette d'expression qui régule l'expression en réponse à une stimulation externe.

4. Méthode selon l'une quelconque des revendications 1 à 3, dans laquelle l'étape d'expression du gène de Tbx6 dans les cellules souches pluripotentes comprend :
une étape d'introduction, dans les cellules souches pluripotentes, d'une cassette d'expression capable d'induire l'expression du gène de Tbx6 en réponse à une stimulation externe ; et
une étape de culture des cellules souches pluripotentes, dans lesquelles la cassette d'expression a été introduite, en présence d'une stimulation externe.

5. Méthode selon l'une quelconque des revendications 1 à 4, dans laquelle l'étape d'expression du gène de Tbx6 dans les cellules souches pluripotentes comprend :
une étape d'introduction, dans les cellules souches pluripotentes, d'une cassette d'expression capable d'induire l'expression du gène de Tbx6 en réponse à une stimulation externe ; et
une étape de culture des cellules souches pluripotentes, dans lesquelles la cassette d'expression a été introduite, en présence d'une stimulation externe et en l'absence de sérum.

6. Méthode selon l'une quelconque des revendications 2 à 5, dans laquelle l'étape de suppression de l'expression du gène de Tbx6 comprend :
une étape de culture des cellules souches pluripotentes, dans lesquelles la cassette d'expression capable d'induire l'expression du gène de Tbx6 en réponse à une stimulation externe a été introduite, en l'absence d'une stimulation externe.

7. Méthode selon l'une quelconque des revendications 1 à 6, dans laquelle les cellules souches pluripotentes sont des cellules ES ou des cellules iPS.

8. Méthode selon l'une quelconque des revendications 1 à 7, dans laquelle le facteur externe est la tétracycline ou la doxycycline.
